# EUROPEAN PATENT APPLICATION

(11) **EP 4 620 479 A1**
(43) Date of publication of application: **24.09.2025**
(21) Application number: 24219570.9
(22) Date of filing: 12.12.2024
(51) Int. Cl.: A61K 35/747, A23L 33/135, A61K 9/00, A61K 31/137, A61K 31/365, A61K 31/7048, A61K 38/12, A61K 38/26, A61P 3/04, A61K 33/00, A61K 33/30

(54) **POSTBIOTIC PREPARATION COMPRISING PEDIOCOCCUS ACIDILACTICI FOR PREVENTING OR TREATING OBESITY**

(30) Priority: 21.03.2024 EP 24382308
(71) Applicant: Genbioma Aplicaciones, S.L., 31110 Noáin, Navarra (ES)
(72) Inventor: AYO MARTÍNEZ, Josune, 48610 URDULIZ (ES); ONECA AGURRUZA, María, 31007 PAMPLONA (ES); ITURRIA GALLEGO, Ignacio, 20001 SAN SEBASTIÁN (ES); YAVOROV DAYLIEV, Deyan, 31600 BURLADA (ES); BARAJAS VÉLEZ, Miguel Ángel, 31001 PAMPLONA (ES); ENCÍO MARTÍNEZ, Ignacio José, 31620 GORRAIZ (ES); ARAÑA CIORDIA, Miriam, 31013 NUEVO ARTICA (ES); CABELLO OLMO, Miriam, 41020 SEVILLA (ES)
(74) Representative: ZBM Patents - Zea, Barlocci & Markvardsen

(57) **Abstract**

The present invention refers to a postbiotic preparation of *Pediococcus acidilactici* (*P. acidilactici),* for use in preventing and/or treating obesity and overweight, in reducing body fat, in preventing fat accumulation, in reducing appetite, in reducing food intake, or in preventing weight gain. The preparation may further comprise zinc, chromium or active ingredients effective in preventing and/or treating obesity, in reducing body fat, in preventing fat accumulation, in reducing appetite, in reducing food intake, in preventing adipogenesis in the adipose tissue, or in preventing weight gain

## Description

This application claims the benefit of European Patent Application EP24382308.5 filed on 21 March 2024.

### Technical Field

The present invention relates to compositions for promoting health, in particular compositions comprising a postbiotic preparation of *Pediococcus acidilacfici* for preventing or treating obesity.

### Background Art

In recent years, there has been a gradual rise in global obesity rates. The World Health Organization (WHO) has characterized the rapid proliferation of obesity as an "infectious disease," coining the term "Globesity."

Obesity escalates the risk of various health issues, leading to complications such as hypertension, hyperlipidemia, cardiovascular disease, sleep apnea, and even cancer. Consequently, obesity diminishes quality of life and may result in premature death, with the life expectancy of morbidly obese individuals significantly lower than that of those with normal weight. While genetic factors may play a role in obesity development, the epidemic is primarily attributed to high-calorie diets and sedentary lifestyles. Lifestyle modifications are, therefore, crucial in preventing obesity and its associated complications.

Although low-calorie diets and regular exercise are traditional methods for weight reduction and obesity treatment, their implementation is challenging, and their effectiveness is limited due to the body's adaptive physiological mechanisms that preserve energy storage. While some drugs like glucagon-like peptide 1 (GLP-1) analogues such as semaglutide and liraglutide, GLP-1/GIP dual agonists such as tirzepatide, gastrointestinal lipase inhibitors such as orlistat, or appetite suppressants such as phentermine or topiramate, have been approved for long-term obesity treatment, they may have side effects, including gastrointestinal disorders. Besides, these are expensive medicaments requiring prescription. Bariatric surgery, which significantly reduces body weight, is available for some individuals, but it is neither accessible nor suitable for many others.

Treatment with products that target the gut microbiota have been proposed as a promising strategy for addressing obesity. These products usually consist of living microbes (probiotics) or substrates that are used by the gut microbiota (prebiotics). However, life microorganisms are hard to manage in an industrial set-up and have limitations related to their shell-life, while prebiotics by themselves seem to have limited efficacy.

Hence, there is a need to develop new strategies for reducing body weight and fat accumulation.

### Summary of Invention

The inventors have found that consumption of a postbiotic preparation of bacteria of the species *Pediococcus acidilactici (P. acidilactici*) or fragments thereof has the surprising effect of preventing weight gain and improving obesity-related parameters with the consequence that these bacteria are useful for preventing and/or treating obesity and overweight or, in general, for reducing appetite, preventing weight gain, preventing fat accumulation and/or reducing body fat.

Thus, in first aspect the invention refers to a postbiotic preparation of *Pediococcus acidilactici* for use in preventing and/or treating obesity or overweight, in reducing body fat, in preventing fat accumulation, in reducing appetite, in reducing food intake, or in preventing weight gain. This aspect can be alternatively formulated as the use of a postbiotic preparation of *Pediococcus acidilacfici* for the manufacture of a medicament for preventing and/or treating obesity or overweight, in reducing body fat, in preventing fat accumulation, in reducing appetite, in reducing food intake, or in preventing weight gain. The invention also refers to a method for preventing and/or treating obesity or overweight, reducing body fat, preventing fat accumulation, reducing appetite, reducing food intake, or preventing weight gain in an animal in need thereof, including a human, comprising administering to said animal in need thereof an effective amount of a postbiotic preparation of *Pediococcus acidilactici.*

As shown in the examples below, a preparation of heat-inactivated *Pediococcus acidilactici* surprisingly was able to prevent weight gain and improved several obesity-related parameters, in particular, the preparation reduced adipocyte size, reduced food efficiency, reduced steatosis, and reduced leptin in mice fed a high-fat diet when compared to controls fed the high-caloric diet but not receiving the inactivated *Pediococcus acidilactici.* Even more surprisingly, the inactivated *Pediococcus acidilactici* preparation (postbiotic preparation) achieved better results in preventing weight gain and improving obesity-related parameters when compared to live *P. acidilactici* (probiotic preparation). A probiotic preparation comprising *Pediococcus acidilactici* was disclosed in WO2021123355 for glycemic control. However, this document does not disclose any effect in controlling obesity. Moreover, the obesity-controlling activity of the postbiotic preparation of the present invention is surprising, since it is usually considered that inanimate bacteria have milder effects than live, metabolically active bacteria. Indeed, WO2021123355 discloses that heat-killed *P. acidilactici CECT* 9879 unexpectedly showed equivalent glycaemia-controlling effects when compared to live cells. The fact that the postbiotic preparation of the present invention shows better obesity-controlling effects when compared to its life counterpart is even more surprising.

A second aspect of the invention refers to a composition comprising a postbiotic preparation of *P. acidilactici* for use in preventing and/or treating obesity or overweight, in reducing body fat, in preventing fat accumulation, in reducing appetite, in reducing food intake, or in preventing weight gain. Again, this aspect may be reformulated as the use of a composition comprising a postbiotic preparation of *P. acidilactici* for the manufacture of a medicament for preventing and/or treating obesity or overweight, in reducing body fat, in preventing fat accumulation, in reducing appetite, in reducing food intake, or in preventing weight gain. The invention also refers to a method for preventing and/or treating obesity or overweight, in reducing body fat, in preventing fat accumulation, in reducing appetite, in reducing food intake, or in preventing weight gain in an animal in need thereof, including a human, comprising administering to said animal in need thereof an effective amount of a composition comprising a postbiotic preparation of *P. acidilactici.*

The postbiotic preparation of the first aspect and the composition of the second aspect possess several advantages when compared with live or metabolically active bacterial preparations while maintaining or even improving their anti-obesity effect. In particular, the postbiotic preparation has better stability, both during industrial processes and storage. This means that, for example, the postbiotic preparation or composition may be subjected to very harsh conditions imposed by conventional industrial processes, such as spray-drying or extrusion, without negatively affecting their activity. The postbiotic preparation also has the advantage of not requiring oxygenation. The postbiotic preparation is also safe. In this sense, it is noted that *P. acidilactici* is generally recognized as safe (GRAS) and thus approved for animal and human consumption. Last but not less, the postbiotic preparation contains inanimate microorganisms, bypassing the problem of acquiring antibiotic resistance genes and virulence factors, which is growing global health threat that requires urgent attention.

The postbiotic preparation of the first aspect, or the composition of the second aspect also have some advantages when compared to drugs for the prevention or treatment of obesity, particularly related to the lack of side effects and the reduced cost. However, it may be convenient to use the postbiotic preparation or composition of the invention in combination with lifestyle changes (e.g., diet modifications) and/or other drugs that have anti-obesity properties in order to obtain a synergistic effect and conveniently reduce the dose of said drug with the subsequent avoidance of side effects caused by them.

### Brief Description of Drawings

**Figure 1****.** Nile Red Staining of pA1c^{®}HI- and pA2cHl- and pA1c^{®}-supplemented worms. (A) % of fat accumulation with respect of the control. (B) % of fat reduction with respect to the control.
**Figure 2****.** ROS production quantification (measured by DHE) of pA1c^{®}HI- and pA1c^{®}-supplemented worms. (A) % of ROS with respect to the control. (B) % of ROS reduction with respect to the control.
**Figure 3****.** Gene expression analysis of key genes involved in the biosynthesis and degradation of lipids, quantified by real-time PCR (qPCR) in C. *elegans.* Gene expression levels were normalized to the housekeeping gene *(pmp-3).* Data are expressed using the 2^{-ΔΔCt} method.
**Figure 4****.** Lifespan analyses of control, probiotic and postbiotic worms. Significance refers to the effect of pA1c^{®}HI with respect to control worms and with respect of probiotic worms (log-rank Mantel-Cox Test, * *p <* 0.05, *** *p* < 0.001).
**Figure 5****.** Nile Red Staining of pA1c^{®}HI-supplemented worms at different temperatures (121-135°C). (A) % of fat accumulation compared to the control. (B) % of fat reduction compared to the control.
**Figure 6****.** Nile Red Staining of the combination pA1c^{®}HI + Chromium-supplemented worms. (A) % of fat accumulation compared to the control. (B) % of fat reduction compared to the control.
**Figure 7****.** Nile Red Staining of the combination pA1c^{®}HI + Zinc-supplemented worms. (A) % of fat accumulation compared to the control. (B) % of fat reduction compared to the control.
**Figure 8****.** Effect of pA1c^{®} and pA1c^{®}HI on (A) body weight gain (%), (B) total weight gain (%)
**Figure 9****.** Effect of pA1c^{®} and pA1c^{®}HI on (A) food intake, and (B) food efficiency. 9-10 animals/group. **p* < 0.05 and "p < 0.01.
**Figure 10****.** Effect of pA1c^{®} and pA1c^{®}HI on (A) adipocyte area and (B) adipocyte diameter. (C) Representative images of H&E-stained adipose tissue sections (scale bars = 100 µm). 9-10 animals/group. ***p* < 0.01.
**Figure 11****.** Effect of pA1c^{®} and pA1c^{®}HI on (A) serum leptin and (B) colonic GLP-1. n = 8-10 animals/group. ^{$$}*p* < 0.01 vs Co; ^{$$$}*p* < 0.001 vs Co; **p* < 0.05; ***p* < 0.01 and ****p* < 0.001. ns: no significant differences.
**Figure 12****.** Impact of pA1c^{®} and pA1c^{®}HI on (A) hepatic and (B) adipose tissue gene expression. n = 8 animals/group. **p* < 0.05, "p < 0.01 and ****p* < 0.001. *Acox:* Peroxisomal acyl-coenzyme A oxidase 1; *Cpt1a:* Carnitine palmitoyl transferase deficiency-type 1; CD36: Cluster of differentiation 36; *Fasn:* fatty acid synthase; ns: no significant differences; *Ppara:* peroxisome proliferator-activated receptor a; *Pparγ:* peroxisome proliferator-activated receptor γ; Srebp: sterol regulatory element-binding protein.
**Figure 13****.** Participants' progression through the intervention.
**Figure 14****.** Changes in anthropometric measurements from baseline to the end of the study (3 months). (A) Total weight, (B) Body mass index (BMI), (C) Fat mass, (D) Visceral fat, (E) Lean mass. Data is expressed as mean ± SEM. *p < 0.05 for postbiotic intra-intervention comparisons.

### Detailed description of the invention

### Definitions

According to the International Scientific Association of Probiotics and Prebiotics (ISAPP), the term "postbiotic" refers to a preparation of inanimate microorganisms and/or their components that confers a health benefit on the host (beyond basic nutrition). "Inanimate microorganisms" is understood as lifeless microorganisms. The word 'components' includes microbial cell components, cell wall components or other structures, as well as microbial metabolites or end products of growth on the specified matrix produced during growth and/or fermentation. "Bacterial metabolites" includes all molecules produced or modified by the bacteria as a result of bacterial metabolism during growth. In the sense of the present invention a "postbiotic preparation" is preferably a preparation comprising or consisting essentially of inanimate *P. acidilacticci* and/or components thereof, preferably comprising or consisting essentially of inanimate (dead) *P. acidilacticci.*

Examples of bacterial metabolites include all organic acids, inorganic acids, bases, proteins and peptides, enzymes and co-enzymes, amino acids and nucleic acids, carbohydrates, lipids, glycoproteins, lipoproteins, glycolipids, vitamins, all bioactive compounds, metabolites containing an inorganic component, and all small molecules, for example, nitrous molecules or molecules containing a sulfurous acid. Thus, a postbiotic preparation may contain whole inanimate cells, lysed cells, or even a cell-free extract obtained from the microbial cells.

The term "probiotic" in the sense of the present invention is defined as a life microorganism that, when consumed in adequate amounts, exerts health benefits to the host beyond inherent basic nutrition.

The term "prebiotics" is generally defined as non-digestible food ingredients that beneficially affect the host by selectively stimulating the growth and/or activity of one or a limited number of bacteria in the colon, and thus improve host health. Typically, prebiotics are carbohydrates (such as oligosaccharides), but the definition does not preclude non-carbohydrates.

"Medicament" as used herein encompasses medicaments for both human and animal usage in human and veterinary medicine. In addition, the term "medicament" as used herein means any substance which provides a therapeutic and/or beneficial effect. The term "medicament" as used herein is not necessarily limited to substances which need marketing approval but may include nutraceuticals and natural remedies.

The term "effective amount" as used herein, means an amount of an active agent high enough to deliver the desired benefit, but low enough to avoid serious side effects within the scope of medical judgment. This term refers to that amount of the active component of the pharmaceutical composition which is sufficient to cause the prevention and/or treatment, of a disease or pathological condition of interest in the mammal, particularly in humans. The effective amount could vary according to the activity of the strain of the invention and its active agents, according to its metabolic stability, in any presentation form, and among other factors, but it can be determined by a person skilled in the art according to his/her own knowledge and this description.

The term "mutants" refer to strains obtained using the bacterial strains of the invention as starting material. A "mutant" of the strain is also understood according to the invention as a "variant" or "derivative".

In this specification the term "preventing" refers to preventing the specified condition from occurring in an animal which may be predisposed to the condition and/or may be exposed to conditions that favor developing of the condition but does not yet experience or display the pathology or symptomatology.

The term "treatment" refers to inhibiting the condition in a mammal that is experiencing or displaying the pathology or symptomatology of the condition (i.e., arresting further development of the pathology and/or symptomatology) or ameliorating the condition in a mammal that is experiencing or displaying the pathology or symptomatology of the condition (i.e., reversing the pathology and/or symptomatology or stopping or slowing its progression). The term "treatment" encompasses prophylactic treatment to prevent the condition, reduce the severity of the condition, or to retard its appearance.

"Obesity" and "overweight" are conditions in which the natural energy reserve, stored in the fatty tissue of animals, in particular humans and other mammals, is increased to a point where it is associated with certain health conditions or increased mortality. The expression "excessive fat accumulation" is understood as having more body fat than is optimally healthy. Obesity and overweight are usually defined by the Body mass index of an individual. "Body mass index" or "BMI" means the ratio of weight in kg divided by the height in meters, squared. "Obesity" is generally defined for a human adult as having a BMI greater than 30. "Overweight" is generally considered for human adults as BMI from 25 to 29.9. For human children above 2 years of age, the BMI is plotted on a BMI vs. age growth chart (for either girls or boys) to obtain a percentile ranking. For children below 2 years (infants) what is plotted is the weight-for-length instead of the BMI. Percentiles and z-scores are the most commonly used indicator to assess the size and growth patterns of individual children. The percentile indicates the relative position of the child's BMI or weight-for-length in a population of individuals with the same sex and age. The z-score indicates the number of standard deviations (SDS) that one individual deviates from the mean of a population with the same sex and age. Human children are considered overweight if their BMI or weight-for-length percentile is located between the 85th and 95th percentile, or the z-score between 1 and 2 SDS. Human children are considered obese if their BMI or weight-for-length percentile is located on or above the 95th percentile, or z-score equal or higher than 2 SDS.

As used herein, the term "functional food" means food which is capable of providing not only a nutritional effect, but is also capable of delivering a further beneficial effect to consumers. Accordingly, functional foods are ordinary foods that have components or ingredients (such as those described herein) incorporated into them that impart to the food a specific functional - e.g. medical or physiological benefit - other than a purely nutritional effect. In the sense of the present invention the term food or functional food includes solid foods as well as beverages.

The term "nutraceutical" means a composition which is capable of providing not only a nutritional effect and/or a taste satisfaction, but is also capable of delivering a therapeutic (or other beneficial) effect to the consumer.

Nutraceuticals cross the traditional dividing lines between foods and medicine.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

In the sense of the present disclosure the expression "consisting essentially of" means that specific further components can be present, namely those not materially affecting the essential characteristics of the composition.

The concentrations disclosed in the present invention are preferably referred to weight percentage ("wt percent" or "% (w/w)"), which represents the percent weight of a single compound relative to the total weight of the composition. However, when the compositions of the invention are in liquid form, the concentrations may refer to % (w/v), i.e. weight/volume percentage. When referring to the concentration of a microorganism in a composition, the concentration is given in CFU/mL or g ("colony forming units" of microorganism per mL or g of total composition). According to the present invention, the bacterial cells in a postbiotic composition or preparation refer to the cells determined in the postbiotic composition or preparation before inactivation. For example, a dose of 10<x> cells in the postbiotic preparation corresponds to 10<x> cfu determined before inactivation. The cfu ("colony forming units") may be determined by methods known in the art.

### Postbiotic preparation

As mentioned above, the first aspect of the invention refers to a postbiotic preparation of *P. acidilactici* for use in preventing and/or treating obesity or overweight, in reducing body fat, in preventing fat accumulation, in reducing food intake, or in preventing weight gain. Attending to the currently accepted definition of "postbiotic", this aspect can be alternatively worded as a preparation comprising or consisting essentially of inanimate *P*. *acidilacticci* cells and/or their components for use in preventing and/or treating obesity, in reducing body fat, in preventing fat accumulation, in reducing appetite, in reducing food intake, or in preventing weight gain.

In one embodiment, the postbiotic preparation comprises inanimate (dead) *P. acidilactici.* Methods to kill bacteria are known in the art. The most common processes used for microbial inactivation are thermal (pasteurization, sterilization, and ohmic heating) and non-thermal (pulsed electric fields, ultrasound, irradiation, and supercritical carbon dioxide) processes. Some methods maintain the structural integrity of the bacterial cells. Non limiting examples of these methods are phenolization, irradiation and formalin or formaldehyde treatment (Fan et at, J Food Prot 2017; Levinson et al., JAMA 1944; Hankaniemi et al, Antiviral Research 2019). One example of killing the bacterial cells without destroying their structural integrity is treating the bacteria with phenol 0.5% (w/v) during 12h at room temperature. In a particular embodiment, the *P. acidilactici* cells have been killed by heat treatment, such as submitting the cells to a temperature from 60 to 120 °C during 1 to 60 minutes, in particular submitting the *P. acidilactici* cells to a temperature from 70 to 90 °C during 20 to 45 minutes, more particularly to a temperature from 75 to 95 °C during 20 to 40 minutes, preferably submitting the *P. acidilactici* cells to a temperature around 80 °C during around 30 minutes. Stirring may be applied during the inactivation (killing) procedure.

In the sense of the present invention, the postbiotic preparation is obtainable by submitting *P. acidilactici* cells to a treatment to kill said cells, such as any of the methods disclosed above (heat, phenolization, etc). In one embodiment, the invention refers to a postbiotic preparation of *P. acidilacfici* for use in preventing and/or treating obesity or overweight, in reducing body fat, in preventing fat accumulation, in reducing food intake, or in preventing weight gain, wherein the postbiotic preparation is obtainable by submitting *P. acidilactici* cells to a treatment to kill said cells, such as any of the methods disclosed above (heat, phenolization, etc).

In one embodiment, the postbiotic preparation comprises *P. acidilactici* cells, wherein at least 70% of the cells are inanimate. In another embodiment, at least 80% of the *P. acidilactici* cells in the postbiotic preparation are inanimate. In a particular embodiment, at least 90% of the *P. acidilactici* cells in the postbiotic preparation are inanimate. In another particular embodiment, at least 95% of the *P. acidilactici* cells in the postbiotic preparation are inanimate. In another particular embodiment, at least 96%, 97%, 98%, or 99% of the P. *acidilactici* cells in the postbiotic preparation are inanimate. In another particular embodiment, 100% of the *P*. *acidilactici* cells in the postbiotic preparation are inanimate.

In another embodiment, the postbiotic preparation comprises a cell lysate of *P. acidilactici.* A cell lysate may be obtained by subjecting the bacterial cells to disruption, for example, by means of a French press. In another embodiment, the postbiotic preparation of the first aspect comprises a cell-free extract of *P*. *acidilactici.* A cell-free extract is obtainable by disrupting the bacterial cells and removing the cell wall and membrane components, for example by centrifugation.

In another embodiment the postbiotic preparation comprises a culture's supernatant of *P. acidilactici.* Said culture's supernatant may be obtainable by culturing *P. acidilactici* in a suitable medium and removing the bacterial cells, for example, by centrifugation. The culture's supernatant contains metabolites secreted by the bacterial cells during their growth, which may have obesity-controlling effects. Said metabolites may be identified, for example, by high performance liquid chromatography (HPLC), matrix-assisted laser desorption/ionization-time-of- flight (MALDI-TOF)-MS, or proton nuclear magnetic resonance (NMR) spectroscopy.

Optionally, the postbiotic preparation may be subjected to a dehydration process. The dehydration can be carried out by a lyophilization process, but the slurry can also be dried by fluidized bed drying, or by atomization, spray-drying, etc. Although the postbiotic preparation is particularly resistant to harsh conditions, including high temperature and/or pressure, cryoprotectants and/or lyophilization additives may be added. The postbiotic preparation may be also be directly frozen. Preservatives such as glycerol may be used when freezing.

The postbiotic preparation according to first aspect of invention may comprise from 10<4>to 10<12> cells/g or ml of total preparation, and more particularly from 10<4>to 10<11> cells/g or ml of total preparation, particularly 10<4>to 10<9> cells/g or ml of total preparation, for example 10<7>, 10<8>, 10<9>, 10<10>, 10<11>, or 10<12> cells/g or ml of total preparation.

### Bacterial strains

The postbiotic preparation described in the present invention is obtained from bacteria that belong to the species *P. acidilactici.* This species is generally recognized as safe (GRAS) and thus approved for animal and human consumption.

In one embodiment, the postbiotic preparation of the first aspect comprises a strain of *Pediococcus acidilactici* deposited at the "Coleccion Española de Cultivos Tipo" (CECT) with identification reference CECT 9879. The strain of *P. acidilactici* CECT 9879 of the invention, was also isolated from cow stool in Navarra region (Spain) and was deposited, according to the Budapest Treaty, on 05.09.2019 in the "Coleccion Española de Cultivos Tipo" (CECT), by the depositor Pentabiol S.L., sited at Poligono Industrial Noain-Esquiroz, Calle S, Nave 4, 31191 Esquiroz, Navarra (Spain). The strain received the accession number CECT 9879 after the International Authority of Deposit declared the strain as viable.

In another embodiment, the postbiotic preparation of the first aspect comprises a strain of *Pediococcus acidilactici* deposited at the "Coleccion Española de Cultivos Tipo" (CECT) with identification reference CECT 9889. The strain of *P. acidilactici* CECT 9889 of the invention, was isolated from cow stool in Navarra region (Spain) and was deposited, according to the Budapest Treaty, on 05.09.2019 in the "Coleccion Española de Cultivos Tipo" (CECT), by the depositor Pentabiol S.L., sited at Poligono Industrial Noain-Esquiroz, Calle S, Nave 4, 31191 Esquiroz, Navarra (Spain). The strain received the accession number CECT 9889 after the International Authority of Deposit declared the strain as viable.

In another embodiment, the postbiotic preparation of the first aspect comprises a strain of
*Pediococcus acidilactici* deposited at the "Coleccion Española de Cultivos Tipo" (CECT) with identification reference CECT 9967, and combinations thereof. The strain of *P. acidilactici* CECT 9967 of the invention, was isolated from cow stool in Navarra region (Spain) and was deposited, according to the Budapest Treaty, on 01.10.2019 in the "Coleccion Española de Cultivos Tipo" (CECT), by the depositor Pentabiol S.L., sited at Poligono Industrial Noain-Esquiroz, Calle S, Nave 4, 31191 Esquiroz, Navarra (Spain). The strain received the accession number CECT 9967after the International Authority of Deposit declared the strain as viable.

The depositor Pentabiol S.L. sited in Poligono Industrial Noain-Esquiroz Calle S, Nave 4, 31191 Esquiroz, Navarra (Spain) authorised GENBIOMA APLICACIONES, S.L. to refer to the deposited biological material in the European patent application having the representative's reference number P6548EP00 and gave their unreserved and irrevocable consent to the deposited material being made available to the public as from the date of filing of this patent application.

In some embodiments, the postbiotic preparation comprises a combination of inanimate cells of *P.acidilactici* and/or their components. In one embodiment, the postbiotic preparation comprises inanimate cells of CECT 9879 and/or its components and inanimate cells of *P.acidilactici* CECT 9889 and/or its components. In other embodiment, the postbiotic preparation comprises inanimate cells of CECT 9879 and/or its components and inanimate cells of *P.acidilactici* CECT 9967 and/or its components. In other embodiment, the postbiotic preparation comprises inanimate cells of CECT 9889 and/or its components and inanimate cells of *P.acidilactici* CECT 9967 and/or its components. In other embodiments the postbiotic preparation further comprises inanimate cells of another *P.acidilactici* strain and/or its components. In other embodiments the postbiotic preparation further comprises inanimate cells of another bacterial strain and/or its components.

An expert in the art will understand that using the bacterial strains of the invention as starting material it is routinely possible to obtain, for example by spontaneous mutation or directed mutagenesis, mutants that retain the characteristics and relevant advantages of the strains of the invention. It would be apparent to a skilled person that such mutant strains may be used in the present invention as obvious alternatives (equivalents) to the strains disclosed herein for obtaining the postbiotic preparation. Methods for obtaining mutants of a given bacterial strain are well known in the art. Examples can be found in (Sambrook, J. and Russell, DW "Molecular Cloning: A Laboratory Manual", Chapter 13, "Mutagenesis", Cold Spring Harbor, 3rd Ed., 2001). Mutants may be obtained by genetic engineering methods, such as site directed mutagenesis. Mutants may also be obtained by subjecting the strains of the invention to mutagenic conditions, such as UV light, or to stress conditions, which may result in obtaining what is usually called "spontaneous mutants".

### Culture

As mentioned above, the postbiotic preparation is obtainable by submitting the bacterial cells to a process for inactivating the same. In any case, the first step to obtain a postbiotic preparation is to obtain the bacterial cells of interest (in the present invention, *P. acidilactici* cells).

The bacterial cells may be obtained by methods well known to a skilled person. For example, a process for preparing a bacterial cell suspension of *P. acidilactici* comprises: (i) inoculating the *P. acidilactici* strain in a culture medium, (ii) subjecting the inoculated culture medium of the step (i) to conditions suitable for growth of *P. acidilactici,* and (iii) optionally subjecting the medium resulting from step (ii) to a concentration step.

Non-limiting conditions suitable for the growth of *P. acidilactici* may be as follows. The bacteria can be cultured on a substrate that may be a liquid broth or solid culture medium, for example in agar plates, using standard culture medium such as Tryptic Soy Agar (TSA), Tryptic Soy Broth (TSB), Man, Rogosa and Sharpe (MRS) agar or broth. Particularly, the culture to be inoculated is in an exponential growth phase close to the stationary phase. Cell multiplication is usually allowed to reach exponential phase, achieving a cell density from 7 × 10 <8> to 2 × 10<10> colony forming units (CFU) / mL or g substrate. Suitable conditions for the growth of the bacteria are temperatures between 30 and 40 °C, for example 35-38 °C, for example 37 °C, and aerobic conditions or microaerophilic conditions (5% of CO₂). Advantageously, the *P. acidilactici* described herein grow to high cell densities in industrial scale several culture media, including lactose-free media and other allergen-free media.

Suitable concentration techniques for the cell suspension obtained as described above are known to the skilled person and may include centrifugation or filtration of the culture. The skilled person may apply the concentration technique to a desired extend such that a concentrated suspension in the culture medium may be obtained or, if desired, the culture medium may be completely removed. By centrifuging the culture, for example, at a minimum of 9000 g, cells may be separated from the culture medium (supernatant). Washing steps may be introduced when required. The cell suspension, concentrated cell suspension or cells may then be used directly to obtain the postbiotic preparation or stored.

Optionally, the bacterial cells or cell suspensions obtained by the methods defined above may be subjected to a dehydration process. The dehydration can be carried out by a lyophilization process, but the slurry can also be dried by fluidized bed drying, or by atomization. The cells may be also be directly frozen or stored as cryoballs.

Moreover, the strains of the invention conveniently grow in different culture media to yield cultures with very high cell density (up to 10<10> cfu/ml) in industrial fermenters without losing their activity. All these features are advantageous for industrial handling and commercial use of the strains.

### Compositions

The postbiotic preparations of the first aspect may be used directly for consumption, for example, for oral consumption, or may be used as an ingredient for preparing a product (for example, an oral composition such as a food product or a pharmaceutical composition). Thus, as mentioned above, in a second aspect the invention provides a composition comprising a postbiotic preparation as defined above for use in preventing and/or treating obesity, in reducing body fat, in preventing fat accumulation, or in preventing weight gain. All embodiments described above for the postbiotic preparation of the first aspect also apply to the composition of the first aspect.

While is it possible to administer the probiotic preparation according to the present invention alone (i.e. without any support, diluent or excipient), the postbiotic preparation is typically administered on or in a support as part of a composition. The composition may be, for example, an oral, parenteral, rectal, vaginal, topical, intradermal, transdermal, subcutaneous, sublingual, intravenous or nasopharyngeal composition. In particular embodiments, the composition of the invention comprises a postbiotic preparation of a *Pediococcus acidilactici* strain selected from the group consisting of CECT 9889, CECT 9967, CECT 9879, and combinations thereof. As mentioned above, this may be alternatively worded as a preparation comprising or consisting essentially of inanimate *Pediococcus acidilactici* selected from the group consisting of the strains CECT 9889, CECT 9967, CECT 9879, and combinations thereof. In one embodiment, the composition comprises a postbiotic preparation of *Pediococcus acidilactici* CECT 9889. In other embodiments, the composition comprises a postbiotic preparation of *Pediococcus acidilactici* CECT 9879. In other embodiments, the composition comprises a postbiotic preparation of *Pediococcus acidilactici* CECT 9967. In other embodiments, the composition comprises a postbiotic preparation of *Pediococcus acidilactici* CECT 9889 and *Pediococcus acidilactici* CECT 9879. In other embodiments, the composition comprises a postbiotic preparation of *Pediococcus acidilactici* CECT 9889 and *Pediococcus acidilactici* CECT 9967. In other embodiments, the composition comprises a postbiotic preparation of *Pediococcus acidilactici* CECT 9879 and *Pediococcus acidilactici* CECT 9967. In other embodiments, the composition comprises a postbiotic preparation of *Pediococcus acidilactici* CECT 9889, *Pediococcus acidilactici* CECT 9879 and *Pediococcus acidilacfici* CECT 9967.

The compositions of the second aspect may comprise from 10<4>to 10<12> cells/g or ml of total composition, and more particularly from 10<5>to 10<11> cells/g or ml of total composition, even more particularly from 10<6>to 10<11> cells/g or ml of total composition, for example 10<7>, 10<8>, 10<9>, 10<10>, or 10<11 > cells/g or ml of total composition. According to the present invention, a dose of 10<x> cells in the composition corresponds to 10<x> cfu determined before inactivation.

In one embodiment, the composition of the second aspect of the invention is an oral composition. In particular embodiments, the oral composition of the invention is selected from the group consisting of a food product, a food ingredient, a food supplement, a pharmaceutical product or a nutraceutical product. These products typically contain additional components (additives) well known to those skilled in the art, such as preservatives, anti-oxidants, emulsifiers, colorants, bulking agents, etc. Appropriate additives are described below. In a very particular embodiment the composition of the second aspect is a food product. Here, the term food is used in a broad sense - and covers food for humans as well as food for animals (i.e. a feed). In a particular embodiment, the food product is for human consumption. In another particular embodiment, the food product is for a non-human animal consumption (feed), in particular for pets or livestock (including fish).

In particular embodiments, the oral composition of the invention is a functional food. In other embodiments the oral composition of the invention is a nutraceutical. In other embodiments the oral composition of the invention is a pharmaceutical composition.

The composition of the present invention may also be used as - or in the preparation of - a pharmaceutical or nutraceutical. Here, the term pharmaceutical or nutraceutical is used in a broad sense - and covers pharmaceuticals or nutraceuticals for humans as well as pharmaceuticals or nutraceuticals for animals (i.e. veterinary applications). In a preferred aspect, the pharmaceutical or nutraceutical is for human use and/or for animal husbandry. When used as - or in the preparation of - a pharmaceutical or nutraceutical composition, the composition of the present invention may be used in conjunction with one or more acceptable excipients or carriers.

The composition of the invention may be presented in any dosage form, for example, solid or liquid, and can be administered by any suitable route. In particular embodiments the pharmaceutical or nutraceutical compositions of the invention are oral compositions (compositions for oral administration). Compositions provided herein suitable for oral administration may be in the form of capsules, cachets, pills, tablets, lozenges, powders, granules, or as a solution or a suspension in an aqueous or non-aqueous liquid, or as an oil-in-water or water-in-oil liquid emulsion, or as an elixir or syrup, or as pastilles.

The tablets, capsules, and other solid dosage forms of the pharmaceutical compositions described herein, such as dragees, pills and granules, may optionally be scored or prepared with coatings and shells, such as enteric coatings and other coatings well known in the art. They may also be formulated so as to provide slow or controlled release of the active ingredient using, for example, hydroxypropylmethyl cellulose in varying proportions to provide the desired release profile, other polymer matrices, liposomes and/or microspheres. Compositions described herein may also be formulated for rapid release, e.g., freeze-dried. These compositions may be formulated to release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Non-limiting examples of acceptable excipients are microcrystalline cellulose, lactose, sodium citrate, calcium carbonate, dibasic calcium phosphate and glycine; disintegrants such as starch, sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc; cryoprotectants, such as DMSO, ethylene glycol, glycerol, 2-Methyl-2,4-pentanediol (MPD), propylene glycol, sucrose and trehalose. Examples of pharmaceutically acceptable carriers include, for example, water, salt solutions, alcohol, silicone, waxes, petroleum jelly, vegetable oils, polyethylene glycols, propylene glycol, liposomes, sugars, gelatin, starch, lactose, amylose, magnesium stearate, talc, surfactants, silicic acid, viscous paraffin, perfume oil, fatty acid monoglycerides and diglycerides, petroethral fatty acid esters, hydroxymethylcellulose, polyvinylpyrrolidone, and the like.

The compositions of the invention may also contain a preservative. Examples of preservatives may include antioxidants, chelating agents, antimicrobial preservatives, antifungal preservatives, alcohol preservatives, acidic preservatives, and other preservatives. The compositions of the invention may also contain an antioxidant. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. The compositions of the invention may also contain a chelating agent. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. The compositions of the invention may also contain a buffering agent. Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminium hydroxide, alginic acid, pyrogen-free water, isotonic saline, Ringer's solution, ethyl alcohol, and combinations thereof.

In one embodiment the composition comprises a lubricant and a bulking agent. the composition of the invention comprises a lubricant, for example, magnesium stearate. The amount of lubricant in the composition may be from 0.1 to 5% of the total composition, or from 0.5 to 2% of the total composition or from 0.5 to 1.5% of the total composition. In another embodiment, the composition comprises a bulking agent, for example selected from cellulose, starch and combinations thereof. The cellulose may be for example microcrystalline cellulose. The starch may be for example corn starch. The amount of bulking agent in the composition may be from 30 to 90% of the total composition, or from 40 to 80% of the total composition, or from 50 to 80% of the total composition, or from 30 to 50% of the total composition, or from 40 to 60% of the total composition. In another particular embodiment the composition comprises a cryoprotectant, for example, sucrose, glycerol or trehalose. The amount of cryoprotectant in the composition may be from 0.5 to 20% of the total composition, or from 1 to 10% of the total composition. In a particular embodiment the composition comprises microcrystalline cellulose, starch and magnesium stearate. In a particular embodiment the composition further comprises a cryoprotectant. By "% of the total composition" is understood herein as % by weight when the composition is solid or % by volume when the composition is liquid. In several embodiments the % is understood as % weight of component/weight of total composition.

In some embodiments, the composition comprises a prebiotic. Examples of suitable prebiotics include complex carbohydrates, complex sugars, resistant dextrins, resistant starch, amino acids, peptides, nutritional compounds, biotin, polydextrose, oligosaccharides, polysaccharide, fructooligosaccharide (FOS), fructans, soluble fiber, insoluble fiber, fiber, starch, galactooligosaccharides (GOS), inulin, lignin, psyllium, chitin, chitosan, gums (e.g. guar gum), high amylose cornstarch (HAS), cellulose, b-glucans, hemi-celluloses, lactulose, mannooligosaccharides, mannan oligosaccharides (MOS), oligofructose-enriched inulin, oligofructose, oligodextrose, tagatose, trans-galactooligosaccharide, pectin, resistant starch, xylooligosaccharides (XOS), locust bean gum, P-glucan, and methylcellulose. Prebiotics can be found in foods (e.g. acacia gum, guar seeds, brown rice, rice bran, barley hulls, chicory root, Jerusalem artichoke, dandelion greens, garlic, leek, onion, asparagus, wheat bran, oat bran, baked beans, whole wheat flour, banana), and breast milk. In particular embodiments the the prebiotic is selected from galacto-oligosaccharide (GOS), fructo-oligosaccharide (FOS), inulin, alginate, xanthan, pectin, locust bean gum (LBG), guar gum, polydextrose (i.e. Litesse^{®}), resistant starch, hydroxypropyl methylcellulose, beta-glucans (derived from example from barely and oat) and arabinoxylan.

In some embodiments the composition comprises a further active ingredient. The further active ingredient may be selected from active ingredients that are effective in preventing and/or treating obesity, in reducing body fat, in preventing fat accumulation, or in preventing weight gain. Non-limiting examples may be selected from the group consisting of Semaglutide, Liraglutide, GLP-1/GIP dual agonists such as tirzepatide, gastrointestinal lipase inhibitor such as Orlistat, appetite suppressants such as phentermine or topiramate, Naltrexone/Bupropion, Setmelanotide, Tirzepatide, fluoxetine, Cagrilintide (an amylin analog), Monlunabant (a CB1 receptor antagonist), and inhibitors of the sodium-glucose cotransporter type 2 (SGLT2 inhibitors) such as canagliflozin, ertugliflozin, dapagliflozin, empagliflozin, and oral formulations of semaglutide, and combinations thereof. In other embodiments, the further active ingredient may be selected from the group consisting of sugar replacers, normoglycemic agents and drugs that control blood sugar levels. In other embodiments the further active ingredient may be one or more different probiotic microorganism, for example, other strains of lactic acid bacteria, including different *P. acidilactici* strains.

In a particular embodiment, the composition comprises a postbiotic preparation of a *Pediococcus acidilactici* strain selected from the group consisting of CECT 9879, CECT 9889, CECT 9967, and combinations thereof, in combination with a further active ingredient selected from semaglutide and Orlistat.

In some embodiments the composition comprises a further ingredient selected from the group consisting of Zinc or a salt thereof, Chromium or a salt thereof (preferably the Chromium is in the form of chromium picolinate), and combinations thereof. In particular embodiment the composition further comprises Zinc. It has been found that the combination of Zinc with heat-inactivated *Pediococcus acidilactici* CECT 9879, CECT 9889, or CECT 9967 has a surprising synergic effect. This is evidenced in example 3 and figure 16. Thus, a composition comprising Zinc and a postbiotic preparation of a *Pediococcus acidilactici* strain selected from the group consisting of CECT 9879, CECT 9889, CECT 9967, or combinations thereof is particularly advantageous. In another embodiment, the composition alternatively or additionally comprises Chromium, in particular Chromium picolinate. It has been found that the combination of chromium picolinate with heat-inactivated *Pediococcus acidilactici* CECT 9879, CECT 9889, or CECT 9967 has a surprising synergic effect.

This is evidenced in example 3 and figure 15. Thus, a composition comprising Chromium and a postbiotic preparation of a *Pediococcus acidilactici* strain selected from the group consisting of CECT 9879, CECT 9889, CECT 9967, or combinations thereof is particularly advantageous. These compositions comprise zinc or chromium in a therapeutic effective amount. In particular, the therapeutic amount of Cr in the composition is an amount appropriate to provide a daily dose of 0.5 to 20 µg, in particular from 2 to 10 µg, more particularly from 4 to 8 µg, for example 6 µg of Cr. In particular, the therapeutic amount of Zn in the composition is an amount appropriate to provide a daily dose of 0.1 to 10 mg, in particular from 0.5 to 5 mg, more in particular from 1 to 2 mg, for example 1.5 mg of Zn. In particular, these compositions may comprise Zinc or chromium and *Pediococcus acidilactici* postbiotic preparation in an amount appropriate to provide a daily dose of 6 µg of Cr or 1.5 mg of Zn and a daily dose equivalent to 10<6>to 10<12> cells *Pediococcus acidilactici.*

### Subjects/Medical Indications

The postbiotic preparation to which the present invention relates is administered to an animal, including for example livestock (including cattle, horses, pigs, sheep, or fish), and humans. In some embodiments the animal is a mammal. In some embodiments the mammal is a companion animal (including pets). In other embodiments the subject is a livestock animal which is not a mammal, for example poultry or fish. In preferred embodiments the subject is a human.

The examples below effectively demonstrate that supplementation with a postbiotic preparation of *Pediococcus acidilactici* has a beneficial effect in preventing weight gain and improving obesity-related parameters. Therefore, the postbiotic preparation of the invention are for use in preventing and/or treating obesity, in reducing body fat, in preventing fat accumulation, or in preventing weight gain. Preventing and treating obesity in the sense of the present invention encompasses preventing or treating overweight and also preventing or reducing excessive fat accumulation. In one embodiment the postbiotic preparation as defined above is for use in preventing obesity. In another embodiment the postbiotic preparation as defined above is for use in treating obesity. Prophylactic treatment is also contemplated. In another embodiment the postbiotic preparation as defined above is for use in reducing body fat. In another embodiment the postbiotic preparation as defined above is for use in preventing fat accumulation. In another embodiment the postbiotic preparation as defined above is for use in preventing weight gain. In another embodiment the postbiotic preparation as defined above is for use in weight control. In another embodiment the postbiotic composition is for use in reducing appetite (in other words for producing satiety or for reducing food intake).

All embodiments described above for the postbiotic preparation of the first aspect or the compositions of the second aspect apply to the medical uses described in this section.

In one embodiment, the postbiotic preparation of the invention is for administering to subjects that are obese or overweight, or subjects that are at risk of being overweight or obese, for example, genetically predisposed individuals, and/or subjects leading a sedentary life and/or that have a high calory intake. In particular embodiments the postbiotic preparation is for administering to subjects that have a high calory intake, i.e. that ingest a high calory diet or an obesogenic diet. The postbiotic preparation is also useful to subjects that have normal weight who do not want to gain weight for aesthetic reasons. The postbiotic preparation is thus useful for weight control strategies. In another embodiment, the postbiotic preparation is for administering to subjects having normal weight.

In some embodiments, administering the probiotic preparation of the invention lowers the weight of the subject. In particular embodiments, the reduction of weight in the subject is of at least 5% relative to a control. In some embodiments the reduction of weight is of at least 10%, of at least 20%, of at least 30%, or of at least 40%, or of at least 50%, or of at least 55%, or of at least 60%, or of at least 65%, or of at least 70%, or of at least 75%, or of at least 80%, or of at least 85%, or of at least 90%, or of at least 95%, or of at least 100%, relative to a control. In some embodiments the reduction of weight is higher that 100% relative to a control. In some embodiments administering the postbiotic preparation of the invention reduces weight in a subject by 10% to 80%, or by 20% to 100%, or by 40% to 120%, or by 50 to 120%, or by 60 to 120%, or by 70 to 120%, or by 80 to 120%, or by 90 to 120%, or by 100 to 120%, or by 50 to 100%, or by 60 to 100% or by 70 to 100% or by 80 to 100% or by 90 to 100%, with respect to a control. In some embodiments the subject is ingesting a hypercaloric diet. In some embodiments the subject is predisposed to having obesity or overweight. In some embodiments, administering the postbiotic preparation of the invention reduces gain weight induced by hypercaloric diet in a subject relative to a control. In some embodiments, the control is the subject before administering of the postbiotic preparation of the invention. In some embodiments, the control is another subject that is not administered the postbiotic preparation. In a particular embodiment the subject is predisposed to having obesity or overweight and is ingesting an hypercaloric diet and the control is the same subject before administration of the postbiotic preparation of the invention or another subject predisposed to obesity or overweight that is ingesting the same hypercaloric diet and is not being administered the postbiotic preparation of the invention.

In some embodiments, the effect in preventing and/or treating obesity, in reducing body fat, in preventing fat accumulation, or in preventing weight gain, or in reducing food intake and/or appetite is achieved by administering the postbiotic preparation of the invention during at least 2 weeks. In other embodiments the postbiotic preparation is administered during at least 4 weeks. In other embodiments the postbiotic preparation is administered during at least 8 weeks. In other embodiments the postbiotic preparation is administered during at least 12 weeks. In other embodiments the postbiotic preparation is administered for at least 6 months. In certain embodiments the postbiotic preparation is administered indefinitely.

While preventing and/or treating obesity (or by preventing excessive fat accumulation, or by preventing weight gain or by reducing body fat), the postbiotic preparation may reduce the risk of suffering from metabolic disorders that are associated with overweight, obesity and/or excessive fat accumulation. Metabolic disorders that are associated with overweight, obesity and/or excessive fat accumulation are well known to skilled artisans. For example, these disorders include cardiovascular diseases such as coronary heart disease; insulin resistance; hypertension; sleep apnoea, respiratory problems and /or dyslipidaemia. In particular, embodiments of the invention the metabolic disorders that are associated with obesity and/or excessive fat accumulation are selected from coronary heart disease, insulin resistance, hypertension, dyslipidaemia, and sleep apnoea.

It is also relevant that the postbiotic preparation of the invention reduced serum makers of liver disease (ALT) as well as fat accumulation in the liver tissue (see examples below). This translates in preventing or reducing steatosis and in preventing liver damage. Thus, in particular embodiments, the postbiotic preparation of the invention is for use in preventing and/or treating steatosis. In other embodiments, the postbiotic preparation is for use in the prevention of liver damage.

The postbiotic preparation of the invention may be used in combination with a further active ingredient. Thus, in one embodiment the invention refers to a postbiotic composition as defined above for use in preventing and/or treating obesity, in reducing body fat, in preventing fat accumulation, or in preventing weight gain, when used in combination with a further active ingredient. This embodiment can be reworded as the use of a postbiotic composition as defined above for the preparation of a medicament for preventing and/or treating obesity, in reducing body fat, in preventing fat accumulation, or in preventing weight gain, in combination with a further active ingredient. The invention also contemplates a method for treating. This aspect can also be formulated as a method for preventing and/or treating obesity, in reducing body fat, in preventing fat accumulation, or in preventing weight gain, the method comprising administering an a postbiotic preparation as defined above, in combination with a further active ingredient.

The further active ingredient is selected, in particular embodiments, from active ingredients that are effective in preventing and/or treating obesity, in reducing body fat, in preventing fat accumulation, or in preventing weight gain. Active ingredients appropriate for use in combination with the postbiotic preparation may be selected from the group consisting of Semaglutide, Liraglutide, GLP-1/GIP dual agonists such as tirzepatide, gastrointestinal lipase inhibitor such as Orlistat, appetite suppressants such as phentermine or topiramate, Naltrexone/Bupropion, Setmelanotide, Tirzepatide, fluoxetine, Cagrilintide (an amylin analog), Monlunabant (a CB1 receptor antagonist), and inhibitors of the sodium-glucose cotransporter type 2 (SGLT2 inhibitors) such as canagliflozin, ertugliflozin, dapagliflozin, empagliflozin, and oral formulations of semaglutide, and combinations thereof.. The use of the postbiotic preparation in combination with these drugs allows for reducing the dose of the drug, thus also limiting their side effects. In a particular embodiment the further active ingredient appropriate for use in combination with the postbiotic preparation may be selected from the group consisting of liraglutide, naltrexone-bupropion, obesity, orlistat, phentermine, phentermine-topiramate, semaglutide, setmelanotide, tirzepatide. In another embodiment the further active ingredient appropriate for use in combination with the postbiotic preparation may be selected from the group consisting of Glucomannan or other viscous fibers, Gymnema sylvestre, Griffonia simplicifolia (5-HTP), Caralluma fimbriata, Conjugated linoleic acid, Garcinia cambogia, Flaxseed, Psyllium Husk, Green Tea, Yerba mate, Chili Peppers, Coffee, Fenugreek, and combinations thereof.

The further active ingredient may also be another bacterial strain, in particular, a further bacterial strain that is effective in preventing and/or treating obesity, in reducing body fat, in preventing fat accumulation, or in preventing weight gain.

The further active ingredient may also be selected from the group consisting of Zinc or a salt thereof, Chromium or a salt thereof (preferably the Chromium is in the form of chromium picolinate), and combinations thereof. As mentioned above, it has been found that the combination of Zinc or Chromium with heat-inactivated *Pediococcus acidilactici* CECT 9879, CECT 9889, or CECT 9967 has a surprising synergic effect.

Non-limiting sugar replacers are xylitol, sorbitol, mannitol, maltitol, lactitol, isomalt, erythritol, D-tagatose, isomaltulose, sucralose or polydextrose, soybean oligosaccharides, isomaltulose (Palatinose^{™}), isomalto-oligosaccharides, gluco-oligosaccharides, xylo-oligosaccharides, manno-oligosaccharides, lactitol, lactosucrose and fructose.

Non-limmiting normoglycemic agents are fructo-oligosaccharide (FOS), inulin, resistant starch, oat and barely beta-glucans, xylitol, sorbitol, mannitol, maltitol, lactitol, isomalt, erythritol, D-tagatose, isomaltulose, sucralose or polydextrose, berberine (main active component of an ancient Chinese herb Coptis chinensis French) and other forms of berberine (Berberine hydrochloride), Avocatin B (fatty acids avocadene and avocadyne and their metabolites), beta-ecdysterone, maqui berry extract (Delphinol^{®}), clove derived polyphenols (Clovinol^{®}), cinnamon extract (CinSulin^{®}), polyphenols from brown seaweeds (InSea2^{®}), natural phenol as resveratrol, flavonoid group of polyphenols as quercetin, gamma linolenic acid (GLA), chromium and other forms of chromium (preferably chromium picolinate, Crominex^{®} 3+, etc), vitamins, minerals, polyphenols, antocianidyns and delphinins, dietary fibers from bamboo shoot shell, Gamma Cyclodextrin, Omega-6 fatty acids, linoleic acid, Guava polysaccharides, zinc, zein, resistant starch, arabinoxylans from wheat, beta-glucans (for example derived from oat or bareley), hydroxypropylmethyl cellulose, pectins, xilitol, sorbitol, manitol, maltitol, lactitol, isomaltose, erythritol, sucralose y polydextrose, D-tagatose, isomaltulose, α-ciclodextrin, fructose, non-digestible hydrocarbons, galacto-oligosaccharide (GOS), in particular alpha-GOS, resistant dextrin, corn stach with high amylose content, corn starch with migh amylose content and Psyllium husk.

Non-limiting examples of drugs that control blood sugar levels include insulin, insulin analogs, biguanides (such as metformin), sulfonylureas (such as carbutamide, chlorpropamide, glibenclamide (Glyburide^{™}), gliclazide, glimepiride, glipizide, gliquidone, tolazamide or tolbutamide), alpha-glucosidase inhibitors (such as acarbose, miglitol or voglibose), thiazolidinediones (TZD) (such as pioglitazone, rivoglitazone or rosiglitazone), meglitinides (such as nateglinide, repaglinide or mitiglinide), dipeptidyl peptidase-4 (DPP-4) inhibitors (such as alogliptin, saxagliptin, sitagliptin or vildagliptin), glucagon-like peptide-1 analogs (such as exenatide, liraglutide, or albiglutide), amylin analogs (such as pramlintide), fast acting insulin analogs (such as insulin lispro, insulin aspart and insulin glulisine), long acting insulin analogs (such as insulin glargine, insulin detemir), dual PPAR agonists (such as aleglitazar) and SGLT2 inhibitors (such as dapagliflozin, remogliflozin and sergliflozin), metformin, α-glucosidase inhibitors, meglitinides, thiazolidinediones, sulphonylureas, biguanidesand acarbose. In one embodiment, the bacteria, compositions or combinations defined above may be used according to the present invention in combination with one or more drugs.

The postbiotic preparation or composition and the further active ingredient are administered concomitantly or separately, in any order, within a therapeutically effective interval.

The postbiotic preparation of the invention may also be conveniently used in combination with a low-calorie diets and/or regular exercise. Thus, one embodiment the invention refers to a postbiotic composition as defined above for use in preventing and/or treating obesity, in reducing body fat, in preventing fat accumulation, or in preventing weight gain, when used in combination with a low-calorie diets and/or regular exercise. This embodiment can be reworded as the use of a postbiotic composition as defined above for the preparation of a medicament for preventing and/or treating obesity, in reducing body fat, in preventing fat accumulation, or in preventing weight gain, in combination a low-calorie diets and/or regular exercise. The invention also contemplates a method for treating This aspect can also be formulated as a method for preventing and/or treating obesity, in reducing body fat, in preventing fat accumulation, or in preventing weight gain, the method comprising administering an a postbiotic preparation as defined above, in combination with a low-calorie diets and/or regular exercise.

### Dosage

In one embodiment, the postbiotic preparation in accordance with the present invention is administered at a dose comprising from 10² to 10¹⁵ cells of *P. acidilactici* per day per animal. In one embodiment, the dose of postbiotic preparation comprises from 10⁵ to 10¹² cells of *P. acidilactici* per day per animal. In another embodiment, the dose of postbiotic preparation comprises from 10⁸ to 10¹¹ cells of *P. acidilactici* per day per animal, in particular, from 10⁹ to 10¹¹ cells of *P. acidilactici* per day per animal, for example, 10⁹, 10¹⁰, or 10¹¹ cells of *P. acidilactici* per day per animal. The cells are inanimate cells and usually refer to the total amount of *P. acidilactici* cells, i.e. if the postbiotic preparation contains *P. acidilactici* CECT 9879, CECT 9889, and CECT 9967 strains, the dose of, for example, 10¹⁰ cells of *P. acidilactici* per day per animal, refers to the sum of *P. acidilactici* CECT 9879, CECT 9889, and CECT 9967 cells. As mentioned above, according to the present invention, a dose of 10^{x} cells in the postbiotic preparation corresponds to 10^{x} cfu determined before inactivation.

When the bacteria, such as a strain of *Pediococcus acidilactici, for example Pediococcus acidilactici* CECT 9889, CECT 9967 or CECT 9879, are used in the present invention together with other bacterial strains, the bacteria may be present in any ratio capable of achieving the desired effects of the invention described herein. Typically, the *Pediococcus acidilactici* to each other or other species ratio is in the range 1:100 to 100:1, suitably 1:50 to 50:1, particularly 1:20 to 20:1, more particularly 1:10 to 10:1, still more particularly 1:5 to 5:1, yet more particularly 1:3 to 3:1 and even more particularly 1:2 to 2:1 and most particularly 1:1.5 to 1.5:1. When combined with Zinc or Chromium, the daily dose of Chromium may be from 0.5 to 20 µg, in particular from 2 to 10 µg, more particularly from 4 to 8 µg, for example 6 µg of Chromium; and the daily dose of Zinc may be from 0.1 to 10 mg, in particular from 0.5 to 5 mg, more in particular from 1 to 2 mg, for example 1.5 mg of Zinc.

For completeness the invention is further described in the following numbered embodiments:
1. A postbiotic preparation of *Pediococcus acidilactici (P. acidilactici)* for use in preventing and/or treating obesity or overweight, in reducing body fat, in preventing fat accumulation, in reducing appetite, in reducing food intake, or in preventing weight gain.
2. A postbiotic preparation of *P. acidilacfici* for use in preventing and/or treating steatosis or for preventing liver damage.
3. The postbiotic preparation for use according to any one of the preceding embodiments, comprising or consisting essentially of inanimate *P. acidilactici* cells.
4. The postbiotic preparation for use according to any one of the preceding embodiments, comprising a lysate of *P. acidilactici* and/or a culture supernatant of *P. acidilactici.*
5. The postbiotic preparation for use according to any one of the preceding embodiments, wherein the P. *acidilactici* is selected from the group consisting of *P. acidilactici* deposited at the "Coleccion Española de Cultivos Tipo" (CECT) with identification reference *CECT* 9879, *P. acidilactici* deposited at the "Coleccion Española de Cultivos Tipo" (CECT) with identification reference CECT 9889, *P. acidilactici* deposited at the "Coleccion Española de Cultivos Tipo" (CECT) with identification reference CECT 9967, and combinations thereof.
6. The postbiotic preparation for use according to any one of the preceding embodiments, wherein the use comprises administering to an animal in need thereof from 10⁵ to 10¹¹ cells of *P. acidilactici* per day per animal, in particular, from 10⁹ to 10¹¹ cells of *P. acidilactici* per day per animal.
7. The postbiotic preparation for use according to any one of the preceding embodiments, wherein the use comprises administering to an animal in need thereof around 10¹⁰ cells of *P. acidilactici* per day per animal.
8. A composition comprising a postbiotic preparation of *P. acidilactici* as defined in any one of embodiments 1-7, for use in preventing and/or treating obesity, in reducing body fat, in preventing fat accumulation, in reducing appetite, in reducing food intake, or in preventing weight gain.
9. A composition comprising a postbiotic preparation of *P. acidilactici* as defined in any one of embodiments 1-7, for use in preventing and/or treating steatosis or for preventing liver damage.
10. The composition for use according to any one of embodiments 8-9, wherein the composition is an oral composition.
11. The composition for use according to the preceding embodiment, wherein the oral composition is a food or feed product, a food or feed ingredient, or a food or feed supplement.
12. The composition for use according to the preceding embodiment, wherein the food or feed product is a functional food or feed product, and optionally further comprises food or feed additives selected from the group consisting of preservatives, anti-oxidants, emulsifiers, colorants, and bulking agents.
13. The composition for use according to any one of embodiments 8-9, wherein the composition is a pharmaceutical or nutraceutical composition and also comprises pharmaceutically acceptable excipients and carriers.
14. The composition for use according to the preceding embodiment, comprising a lubricating agent, and/or a bulking agent, and/or an enteric coating.
15. The composition for use according to any one of embodiments 8-14, comprising from 10<2>to 10<15> cells/g or ml of total composition, in particular from 10<7>to 10<11> cells/g or ml of total composition, preferably from 10<10>to 10<11> cells/g or ml of total composition and, optionally, further comprises zinc or chromium, such as chromium picolinate, preferably in an amount appropriate to provide a daily dose from 2 to 10 µg, preferably of around 6 µg of Cr, or from 0.5 to 5 mg, preferably of around 1.5 mg of Zn.
16. The postbiotic preparation for use according to any one of embodiments 1-7 or the composition for use according to any one of claims 8-15, that is administered to subjects consuming a hypercaloric diet.
17. The postbiotic preparation for use according to any one of embodiments 1-7 or 16 or the composition for use according to any one of claims 8-15 or 16, that is administered to an animal.
18. The postbiotic preparation or the composition for use according to the preceding embodiment, wherein the animal is selected from a human, a pet, or life-stock (including cattle and fish).
19. The postbiotic preparation for use according to any one of embodiments 1-7 or 16-18 or the composition for use according to any one of claims 8-15 or 16-18, when used in combination with a further active ingredient that is effective in preventing and/or treating obesity, in reducing body fat, in preventing fat accumulation, in reducing appetite, in reducing food intake, in preventing adipogenesis in the adipose tissue, or in preventing weight gain.
20. The postbiotic preparation or composition for use according to the preceding embodiment, wherein the further active ingredient is selected from the group consisting of a probiotic microorganism, including P. *acidilactici* strains, Semaglutide, Liraglutide, GLP-1/GIP dual agonists such as tirzepatide, gastrointestinal lipase inhibitor such as Orlistat, appetite suppressants such as phentermine, topiramate, or natural appetite suppressants, Naltrexone/Bupropion, Setmelanotide, Tirzepatide, and combinations thereof.
21. The postbiotic preparation for use according to any one of embodiments 1-7 or 16-18, or the composition for use according to any one of claims 8-15 or 16-18, when used in combination with (a) a further ingredient selected from the group consisting of Zinc or a salt thereof, Chromium or a salt thereof, such as chromium picolinate, and combinations thereof.
22. The postbiotic preparation or composition for use according to any one of embodiments 19-21, wherein the postbiotic preparation or composition and the further (active) ingredient are administered concomitantly or separately, in any order, within a therapeutically effective interval.
23. The postbiotic preparation for use according to any one of embodiments 1-7 or 16-18, or the composition for use according to any one of claims 8-15 or 16-18, when used in combination with a low-calorie diet and/or regular exercise.
24. The postbiotic preparation for use according to any one of embodiments 1-7 or 16-23 or the composition for use according to any one of claims 8-15 or 16-23, comprising *P. acidilactici CECT 9879.*
25. The postbiotic preparation for use according to any one of embodiments 1-7 or 16-24 or the composition for use according to any one of claims 8-15 or 16-24, comprising *P. acidilactici CECT* 9889*.*
26. The postbiotic preparation for use according to any one of embodiments 1-7 or 16-24 or the composition for use according to any one of claims 8-15 or 16-24, comprising *P. acidilactici CECT* 9967*.*
27. The postbiotic preparation for use according to any one of embodiments 1-7 or 16-24 or the composition for use according to any one of claims 8-15 or 16-24, comprising *P. acidilactici CECT* 9879, *P. acidilactici CECT* 9889, *and P. acidilacfici CECT 9967.*
28. A postbiotic preparation or composition for use according to any one of the preceding embodiments, wherein the postbiotic preparation is obtainable by submitting *P. acidilactici* cells to a treatment to kill or inactivate said cells.
29. A postbiotic preparation for use according to the preceding embodiment wherein the treatment to kill or inactivate the cells comprises a treatment selected from the group consisting of thermal inactivation, such as pasteurization, sterilization, or ohmic heating, pulsed electric fields, ultrasound, irradiation, supercritical carbon dioxide treatment, phenolization, formalin treatment, and formaldehyde treatment.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### Example 1: C. elegans model

### 1. Materials and methods

### 1.1. Strains and culture

The postbiotic pA1c^{®}HI *Pediococcus acidilactici* CECT 9879, postbiotic pA2cHl *Pediococcus acidilactici* CECT 9889 and probiotic pA1c^{®} *Pediococcus acidilactici* CECT 9879, were used at a concentration of 5×10⁶ cells/mL and 5×10⁶ CFU/mL respectively for all the assays. All postbiotic preparations were obtained by heat-inactivation of the corresponding *P. acidilactici* strains. The *Caenorhabditis elegans* (C. *elegans)* strain used were: N2 Bristol as wild-type strain. The strain was obtained from the Caenorhabditis Genetics Center (CGC, University of Minnesota, Minneapolis, MN, USA).

C. *elegans* was cultured on nematode-growth-medium (NGM) at 20 °C. *Escherichia coli* OP50 (E. *coli,* grown in LB Broth Lennox at 37 °C) was utilized as standard nematode sustenance.

### 1.2. Experimental design

All tests were carried out in quadruplicate, in six-well cell culture plates with 4 mL NGM or with 4 mL glucose-loading (10 mM) NGM (NGMg) per well (with the pA1c^{®}, the pA1c^{®}HI and CECT 9889 pA2cHl spread or not inside the medium). Glucose was added to the medium with the aim of mimicking hypercaloric and obese conditions.

Plates with Orlistat (1.5 mg/mL, Sigma Aldrich, St. Louis, MO, USA) were used as a positive control of fat accumulation reduction based on previous studies in where this drug for human use (pancreatic lipase inhibitor) was also used as positive control. All experiments were performed at the concentration of 5 × 10⁶ cells/mL CFU/mL (colony-forming unit/millilitre of water) of pA1c^{®}HI, pA2cHl and or CFU/mL (colony-forming unit/millilitre of water) of pA1c^{®}. After pA1c^{®}HI, pA2cHl and pA1c^{®} were added to NGM, plates were allowed to solidify and dry in the dark to protect them from light oxidation.

Thereafter, 150 µL of an overnight culture of *E. coli* OP50 were seeded, and plates were again incubated until dried at room temperature in the dark. For all assays, gravid animals were subjected to a standard hypochlorite treatment to obtain age-synchronized worms (wild-type or mutants). The eggs were allowed to hatch overnight in M9 medium, and about 2000 L1 larvae were transferred onto plates and grown until L4 or one-day adult stage.

### 1.3. Nile Red Staining

Nile Red staining is a dye for neutral lipids and is useful for the quantification of the fat accumulation of the worm. Briefly, L4 worms grown in NGM or NGMg with different treatments (control, orlistat, pA1c^{®} HI, pA2cHl and pA1c^{®},) were collected in 1.5 mL tubes and washed three times with PBST (0.01% of Triton X-100 in phosphate-buffered saline). Then, the worms were put on ice for 15 min and fixed in 40% isopropanol for 3 min. Staining was carried out by adding 150 µL of Nile Red solution (3 µg/mL) per tube and incubating (30 min) with moderate shaking at room temperature in the dark. After that, the worms were washed in PBST and mounted on a 2% agarose pad for microscopy visualization.

### 1.4. ROS quantification

Fluorescent dye dihydroethidium (DHE; Dihydroethidium BioReagent, ≥95% (HPCE), Sigma-Aldrich, St. Louis, MO, USA) was used to quantify ROS levels *in vivo.* Briefly, 750 synchronized L1 larvae were transferred into NGMg plates holding water (control group), pA1c^{®} or pA1c^{®}HI, and were allowed to grow in them until they reached L4. Once they reached that stage, L4 worms were harvested, washed with PBST, and incubated in a 3 µM DHE solution (in PBS) during 30 min. Subsequently, worms were washed with PBST and mounted on a 2% agarose pads with a 1% of sodium azide.

### 1.5. Image acquisition and quantification

For all conditions tested, approximately 300 animals were fixed and stained. Images of Nile Red assay were taken under the same conditions (calibration: 0.68 µm/px; optics: SHR Plan Apo 1×; numerical aperture: 0.15; refractive index: 1; format: 1280 × 960 2 × 2 Binning; exposure: ME 800 ms (-2.0 EV); Analog Gain: 2.00; metering mode: average; BithDepth: 8; zoom: 10×. Fluorescent images of Nile Red stained worms were captured at 10× magnification on a Nikon SMZ18 research stereomicroscope equipped with an epi-fluorescence system and a DS-FI1C refrigerated color digital camera (Nikon Instruments Inc., Tokyo, Japan). The dihydroethidium (DHE)-labeled ROS formation was detected by measuring the fluorescence intensity using a Nikon Eclipse 80i epi-fluorescent microscope, equipped with a TRITC filter (Ex 540-625; DM 565; BA 605-655) (Nikon Instruments Inc., Tokyo, Japan).

Images were taken at the same conditions and integration time under a GFP filter (Ex 480-500; DM 505; BA 535-550).

In all cases, the image analysis was performed using ImageJ v1.53e software. The mean value, calculated as the fluorescence mean value per pixel, together with the integrated density and the volume of the worms, were determined. Approximately 25-40 worms were examined in four independent experiments for each condition.

### 1.6. Egg lying and development

To ensure that the supplementation with the bacterium did not affect the development of the worm, egg lying was observed in young adult nematodes (day 3 of growth) grown on NGMg agar plates supplemented or not with pA1c^{®} HI and pA1c^{®}. The images were taken directly on the plates at 135× magnification using a Nikon SMZ18 stereomicroscope equipped with a Nikon DS-Fi1C high-definition colour camera (Nikon Instruments Inc., Tokyo, Japan).

### 1.7. Lifespan Analyses

Synchronized L1 larvae were transferred to NGM or NGMg plates containing water (control group), pA1c^{®} or pA1c^{®}HI for 46 h, to allow C. *elegans* to develop to L4 stage. Four replicates were used per condition. At least 50-65 L4 larvae per replicate were transferred then onto new plates containing the same treatment that worm has been exposed to +40 µM of 5-fluoro-2-deoxyuridine (FUDR, #856657, Sigma-Aldrich, St. Louis, MO, USA). Surviving or dead animals were counted daily, until all nematodes died. Worms were scored as dead when they failed to respond to gentle touch with a platinum wire.

### 1.8. Gene expression analyses

For gene expression analyses, synchronized L1 wild-type worms were exposed to NGM and NGMg treated with 5 × 10⁶ cells/mL of pA1c^{®}HI, 5 × 10⁶ CFU/mL of pA1c^{®}, or non-treated NGM and NGMg in eight biological replicates. Trizol^{®} RNA isolation reagent (Thermo Fisher Scientific, Paisley, UK) was used to extracted total RNA from C. *elegans* N2 strain according to the manufacturer's instructions. NanoDrop ND-1000 spectrophotometer (Thermo Fisher Scientific, Wilmington, DE, USA) was used to determined concentration and purity of RNA at 260/280 nm. Afterwards, 1000 ng of RNA was treated with DNAse (AmbionTM DNase I, RNase-free; Thermo Fisher Scientific Inc., Waltham, MA, USA) according to the manufacturer's protocol. For the quantitative gene expression analyses, DNA-free RNA was reverse-transcribed into cDNA. Gene expression analyses were performed by quantitative real-time PCR (qPCR) using the TaqMan Universal PCR master mix and specific probes from Applied Biosystems Technologies (Thermo Fisher Scientific Inc., Waltham, MA, USA) and Integrated DNA Technologies Inc. (Coralville, IA, USA). All reactions were performed using a CFX384 TouchTM Real-Time PCR Detection System (BioRad, Hercules, CA, USA). The expression level of each gene was normalized compared to the expression of the *pmp-3* gene from Life Technologies (TaqMan Gene Expression Assays, Carlsbad, CA, USA), which were used as housekeeping gene control. Gene expression differences between treated and untreated worms were quantified using the relative quantification 2^{-ΔΔCt} method.

### 1.9. Statistical analysis

C. *elegans* body fat reduction (Nile Red) between intervention and control condition (NGM group), together with oxidative stress (DHE), and real-time PCR data were evaluated by the one-way ANOVA test followed by the Student-Newman-Keuls (SNK) multiple comparisons test when statistical significance (p < 0.05) was reached in the ANOVA test. For lifespan assays, log-rank (Mantel-Cox test) between groups was performed. All tests were performed using GraphPad Prism v. 9.

### 2. Results

### The postbiotic Pediococcus acidilactici reduces fat accumulation in C. elegans with effectiveness comparable (p>0.05) to anti-obesity drugs

C. *elegans,* a free-living nematode, is an experimental *in vivo* model that has been extensively employed in the study of obesity due to its favourable features such as its compact size, short life cycle, large brood size, easy handling, low cost, availability of complete genetic information, 65% conserved human diseases-associated genes and relatively easy genetic manipulation (Yue *et al.,* 2021). The measurement of fat storage and lipid droplets in C. *elegans* is possible by the visualization under microscopy of fat-soluble dyes such as Nile Red (Escorcia *et al.,* 2018).

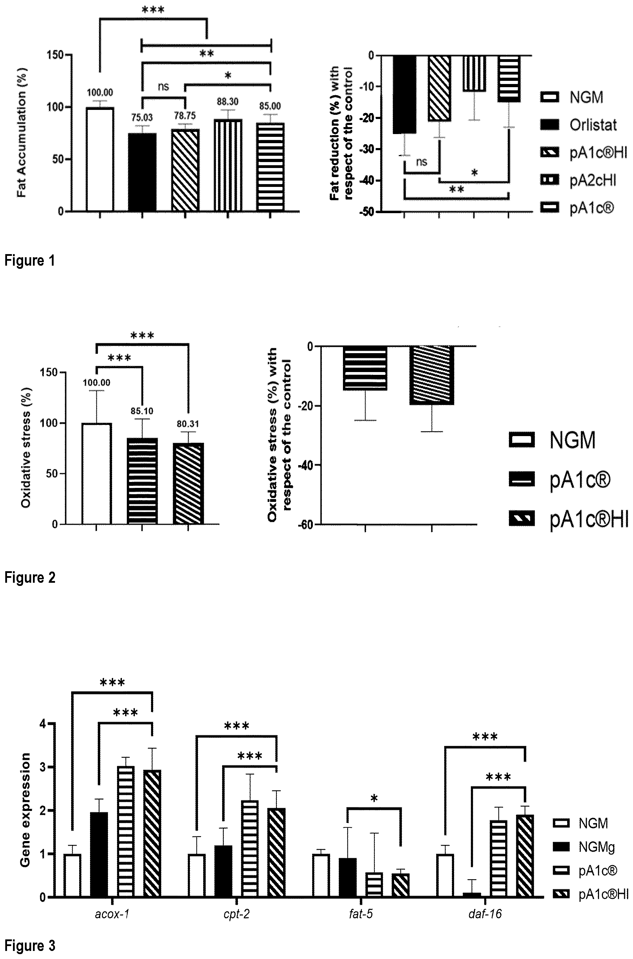

The following results were obtained in an experiment with a sample size of 80 worms per group, ensuring robust and reliable data. At a dose of 5×10⁶ cells/mL, pA1c^{®}HI significantly reduced fat accumulation in wild-type C. *elegans* by 21.2±5.0% compared to control worms (p < 0.001) (Figure 1), while at a lower dose of 5×10⁵ cells/mL, fat reduction was 19.3±4.3% (data not showed). Remarkably, the fat-reducing efficacy of pA1c^{®}HI at 5×10⁶ cells/mL was comparable to that of the anti-obesity drug Orlistat (25±7.0 fat reduction), as no significant differences were observed between them (p > 0.05) in comparison with control worms (Figure 1). Surprisingly, this finding highlights the potential of pA1c^{®}HI as a natural postbiotic for fat reduction, offering similar effectiveness to that of well-established anti-obesity medications.

Additionally, pA1c^{®}HI demonstrated greater efficacy in reducing fat accumulation compared to the probiotic pA1c^{®}. At the same dose of 5×10⁶ cells/mL, pA1c^{®}HI reduced fat storage by 21.2±5.0%, whereas worms supplemented with 5×10⁶ CFU/mL of pA1c^{®} showed only a 15.0±8.0% reduction in fat (p < 0.05) (Figure 1). This statistically significant differences between the postbiotic and the probiotic underscores the superior anti-obesogenic activity of the postbiotic form over its probiotic counterpart, suggesting that surprisingly, postbiotics like pA1c^{®}HI may represent more effective alternatives for managing lipid accumulation. Furthermore, the postbiotic *Pediococcus acidilactici* CECT 9879 (pA2cHI) also demonstrated fat-reducing activity. At a dose of 5×10⁶ cells/mL, pA2cHl significantly reduced lipid storage in C. *elegans* by 12±9.0% compared to control worms (p < 0.001) (Figure 1). This variability suggests that in some cases, pA2cHl achieved reductions exceeding 20%, comparable to the results observed with pA1c^{®}HI. These results reinforce the value of the *Pediococcus acidilactici* species as a promising agent in the fight against obesity, supporting its potential as a natural solution for managing fat accumulation.

Taken together, surprisingly, the results demonstrate that the postbiotic pA1c^{®}HI is highly effective in reducing fat accumulation in C. *elegans* (up to a 26% of fat reduction), achieving effects comparable to the anti-obesity drug Orlistat (no statistical difference between them, p > 0.05) and outperforming the probiotic pA1c^{®} (statistical differences between them, p < 0.05). Additionally, pA2cHl also showed potential improving the response of the lipid metabolism in C. *elegans,* highlighting the promise of *Pediococcus acidilactici* as a valuable species for the prevention and treatment of obesity-related conditions

Moreover, the fat-reducing activity of pA1c^{®}HI did not present any detrimental effect on the organism's growth and correct development. Comparing pA1c^{®}HI-supplemented worms with control and pA1c^{®}-supplemented worms at 72h, it was observed that all the groups exhibited the presence of eggs and L1 larvae as expected, with no differences in their time of appearance. This shows that pA1c^{®}HI is a safe product and that the lipid accumulation reduction is not accompanied by toxicity or harmful effects on the organism's growth and development.

Oxidative stress plays a crucial role in fat accumulation in C. *elegans,* as the buildup of reactive oxygen species (ROS) can lead to cellular damage and metabolic disruptions, promoting fat storage. Therefore, oxidative stress is strongly related to worm fat accumulation, and often a reduction in one can lead to a reduction in the other. At a dose of 5×10⁶ cells/mL, the postbiotic significantly reduced reactive oxygen species (ROS) accumulation in C. *elegans* by 19.7±9.8% compared to the control group (p < 0.001) (Figure 2). In contrast, the live probiotic reduced ROS levels by only 14.9±12.1% at the same dose (p < 0.05). These surprisingly findings suggest that the postbiotic is more effective than the probiotic in enhancing the response to oxidative stress, which as it was said above, is closely linked to fat accumulation and obesity. The ability of the postbiotic to significantly lower ROS levels highlights its potential as an effective strategy not only for reducing oxidative stress but also for combating obesity and related metabolic disorders by mitigating the underlying cellular damage caused by ROS.

Regarding gene expression analyses, in C. *elegans,* several key genes regulate lipid metabolism and fat storage, with *daf-16, acox-1, cpt-2,* and fat-5 playing central roles. The *daf-16* gene, a crucial transcription factor, is involved in controlling fat accumulation and mobilization. Activation of *daf-16* enhances fat utilization and reduces lipid storage, while its impaired function can lead to excessive fat accumulation, contributing to obesity. Similarly, the acox-1 gene encodes an enzyme involved in the initial steps of peroxisomal fatty acid oxidation, promoting the breakdown of fats for energy production. cpt-2 regulates the transport of fatty acids into mitochondria for oxidation, further supporting fat utilization. The fat-5 gene, which encodes a desaturase enzyme, influences the composition of fatty acids, affecting fat storage and membrane fluidity. Together, these genes coordinate lipid metabolism, and their dysregulation can lead to an imbalance in fat storage, promoting obesity. Figure 3 shows that the postbiotic significantly increased (20 times) the fat utilization and lipid breakdown *(daf-16)* and in a 50% the peroxisomal (*acox*-1) and mitochondrial (*cpt-2*) beta-oxidation, in addition to inhibiting (>50%) fatty acid biosynthesis (*fat-5*) compared to high-fat diet control worms, giving some light to the biomolecular mechanism of action by which the postbiotic surprisingly is able to reduce fat accumulation.

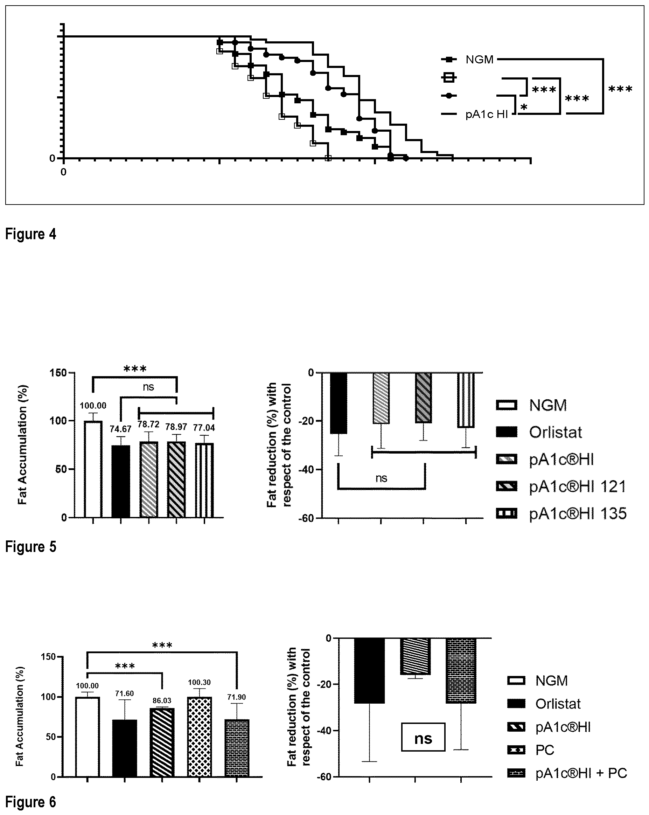

In *C*. *elegans,* the regulation of lipid metabolism is closely linked to both lifespan and aging, with obesity playing a significant role in these processes. Increased fat accumulation, often associated with impaired lipid metabolism, has been shown to shorten the lifespan of *C*. *elegans.* This is because excessive lipid storage can lead to oxidative stress, cellular damage, and reduced physiological function, all of which accelerate aging; therefore, improved lifespan leads to improved lipid metabolism, which in turn may contribute to a reduction in fat accumulation. In this line, At a dose of 5×10⁶ cells/mL, the postbiotic significantly increased the life expectancy of *C*. *elegans* by 23.5% compared to the control group (*p* < 0.001), 30% compared to the high-fat diet control (*p* < 0.001), and 6% compared to the probiotic group (*p* < 0.05) (Figure 4). This demonstrates that the postbiotic not only improves the lifespan of the worms but also counteracts the negative effects of a high-fat diet. Remarkably, the postbiotic's effect on longevity was significantly higher than that observed in the probiotic group, indicating that the postbiotic form is more effective in promoting healthspan. These findings highlight the potential of the postbiotic as a therapeutic agent for enhancing lifespan and mitigating the aging effects associated with obesity.

On the other hand, at the same dose, the postbiotic subjected to 121°C and 135°C for 30 minutes significantly reduced fat accumulation in *C*. *elegans* by 21-23% compared to the control group (*p* < 0.001) (Figure 5). Notably, no statistically significant differences were observed between these temperature-treated postbiotics and the positive control, Orlistat, indicating that these heat-treated-postbiotic are as effective as this anti-obesity drug in reducing fat storage. Furthermore, the trial demonstrated that the postbiotics exposed to these high temperatures retained the same efficacy as the untreated postbiotic. These findings highlight the stability and strong fat-reducing potential of the postbiotic, even after prolonged heat treatment, positioning it as a robust natural alternative to pharmaceutical interventions like Orlistat. Moreover, The remarkable ability of postbiotics to retain their fat-reducing efficacy even after being subjected to extreme temperatures, such as 121°C and 135°C for 30 minutes, underscores their potential as a robust and versatile tool in combating obesity. Unlike probiotics, which often require strict storage and handling conditions to maintain viability, postbiotics demonstrate exceptional stability, allowing them to be incorporated into a wide range of food matrices, including those subjected to high-temperature processes such as baking, cooking, or sterilization. This surprising resilience not only broadens the scope of applications for postbiotics in functional foods and dietary interventions but also enhances their shelf life and ease of transportation. Their consistent efficacy in reducing fat accumulation, even after exposure to such high-temperature treatments, positions postbiotics as a superior alternative to probiotics, offering both practical advantages and potent bioactive benefits for promoting health and addressing obesity-related challenges.

### Example 2: C. elegans model; Synergistic Effects of Postbiotic pA1cOHl Combined with Chromium Picolinate and Zinc on Fat Reduction

### 1. Materials and methods

### 1.1. Strains, culture, experimental design and statistical methods

The postbiotic pA1c^{®}HI *Pediococcus acidilactici* CECT 9879 was as in example 1. The postbiotic was combined with chromium picolinate (PC) (10 mM) or Zinc (Zn) (10mM), by dissolving the substances in the NGM media during media preparation. All postbiotic preparations were obtained by heat-inactivation of the corresponding *P. acidilactici* strains. The *Caenorhabditis elegans* (*C*. *elegans)* strains and culture were also as in example 1.

The experimental design of the following experiments was the same as in example 1, with the pA1c^{®}HI, the PC and the Zn spread or not inside the medium. The statistical analyses were also the same.

### 1.2. Nile Red Staining

Nile Red staining assay was carried out in the same way as example 1. In this case, with 6 different treatments (control, orlistat, pA1c^{®}HI, pA1c^{®} HI + PC, pA1c^{®} HI + Zn and pA1c^{®}).

### 2. Results

### The combination containing postbiotic Pediococcus acidilactici with chromium picolinate or zinc reduced fat accumulation in C. elegans

At a dose of 5×10⁶ cells/mL, the postbiotic significantly reduced fat accumulation in *C*. *elegans* by over 14% compared to the control group (*p* < 0.001) (Figure 6). Furthermore, the combination of the postbiotic with Chromium Picolinate (PC) enhanced the fat-reducing effect, achieving a 29% reduction in fat accumulation. Notably, no statistically significant differences were observed between either the postbiotic alone or the postbiotic-PC combination and the positive control, Orlistat, indicating that both approaches are as effective as this anti-obesity drug.

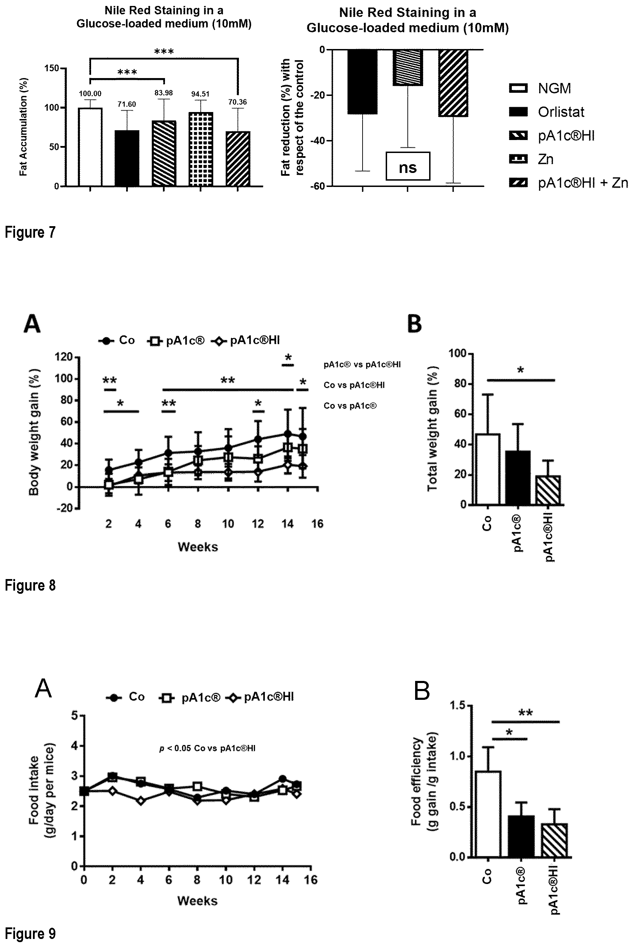

Additionally, the combination of the postbiotic with Zinc (Zn) further enhanced the fat-reducing effect, achieving a 30% reduction in fat accumulation (Figure 7). As in the case of PC, no statistically significant differences were observed between either the postbiotic alone or the postbiotic-Zn combination and the positive control, Orlistat, indicating that both approaches are as effective as this anti-obesity drug.

The conclusions drawn from the last two paragraphs emphasize the synergistic potential of combining postbiotics with specific micronutrients like Chromium Picolinate and Zinc. These combinations significantly enhance the fat-reducing effects of postbiotics, achieving reductions comparable to the anti-obesity drug Orlistat. This synergy not only underscores the versatility of postbiotics but also highlights their potential as a natural, effective, and safe alternative for addressing obesity when paired with targeted dietary supplements.

### Example 3: murine model

### 1. Materials and Methods

### 1.1. Experimental design

Thirty male C57BL/6 mice (Charles River Laboratories) aged 9 weeks were acclimated for 2 weeks with a standard chow diet. The animals were randomly divided and allocated into three groups (n = 10 each): (1) Control group (Co), animals receiving a high-fat diet (HFD); (2) animals receiving HFD plus a probiotic formulation (pA1c^{®} *Pediococcus acidilactici* CECT 9879, prepared as explained below), and (3) animals receiving HFD plus a postbiotic formulation with heat-inactivated (HI) (pA1c^{®})-HI *Pediococcus acidilactici* CECT 9879, prepared as explained below).

During the study, all groups had *ad libitum* access to the diets and water. To verify food acceptance, food and drink intake were visually confirmed by one researcher throughout the experimental study. Food intake was monitored every 2 weeks. At the end of the study (15 weeks), all animals were sacrificed by cervical dislocation for the collection tissue samples (liver, adipose tissue and colon) and blood. Tissue samples for quantitative real-time PCR were cut and frozen at -80 °C immediately after collection. Tissue samples for histological analysis were washed with PBS and fixed in a formaldehyde solution (10%) for 24 h. Blood samples were centrifuged for 8 min at 2000 rpm, and serum samples were stored at -80 °C until analysis. Stool samples were collected at the end of the study for the analysis of the faecal microbiota through 16S rRNA sequencing. All animal procedures were performed under protocols approved by the Institutional Committee on Care and Use of Laboratory Animals (CEEA, University of Navarra) (Protocol number: CEEA/017-20).

### 1.2. Diets and postbiotic preparation

The probiotics/postbiotics were mixed with the HFD every 2 weeks under sterile conditions and kept at 4 °C until use. The HFD (TD.06414, Envigo, Tekla, USA) contains 60% of kcal from fats (detailed information in table 1).

**Table 1. Nutritional information TD.06414 (Envigo)**

| Formula | g/Kg | Composition | % by weight | % kcal from |
|---|---|---|---|---|
| Lard | 310.0 | Fat | 34.3 | 60.3 |
| Casein | 265.0 | Carbohydrate | 27.3 | 21.4 |
| Maltodextrin | 160.0 | Protein | 23.5 | 18.3 |
| Sucrose | 90.0 | Kcal/g | 5.1 | |
| Cellulose | 65.5 | | | |
| Mineral Mix, AIN-93G-MX (94046) | 48.0 | | | |
| Soybean Oil | 30.0 | | | |
| Vitamin Mix, AIN-93-VX (94047) | 21.0 | | | |
| L-Cystine | 4.0 | | | |
| Calcium Phosphate, dibasic | 3.4 | | | |
| Choline Bitartrate | 3.0 | | | |
| Blue Food Color | 0.1 | | | |

| | | | | |
|---|---|---|---|---|
| Information obtained from Envigo Teklad Diets | | | | |

The dose of the pA1c^{®}HI in the diet was adjusted to cells of inactivated *Pediococcus acidilactici* CECT9879 per day/animal. Before the study, pA1c^{®}HI counting in HFD was determined to confirm that the animals received the total cells of *Pediococcus acidilactici* during the intervention.

The dose of the pA1c^{®} in the diet was adjusted to 1 × 10¹⁰ CFU of *Pediococcus acidilactici* CECT9879 per day/animal, and the microbiological load in the diet was confirmed using the classic plate count method (37 °C and 5% CO₂ during 48 h). Before the study, pA1c^{®} survival in HFD was determined to confirm that animals received viable microorganisms during the intervention.

### 1.3. Body weight, food efficiency

Body weight (BW) was recorded every 2 weeks. Food efficiency was determined as grams of weight gain /grams of food intake between the control and treated animals.

### 1.4. Histological analysis

Fixed tissue samples (liver and adipose tissue) were embedded in paraffin and cut into 3 µm thick sections. Sections were stained with hematoxylin and eosin (H&E) for light microscopy examination.

The villus length was analyzed in H&E-stained sections from the small intestine. Five longest villi per slide were measured in twelve different sections of each animal. In PAS-stained small intestine sections, the percentage of goblet cells per total enterocytes was calculated in ten random areas per animal.

For immunohistochemical analysis, sections were dewaxed in xylene and rehydrated through a graded alcohol series. Endogenous peroxidase activity was blocked by placing sections in 3% hydrogen peroxidase for 10 min. For GLP-1 immunolabelling, microwave antigen retrieval was carried out with EDTA buffer (1 mmol/L, pH 8) for 30 min. Nonspecific binding sites were blocked with 5% goat normal serum in TBS-Tween (Wash buffer, Dako, Glostrup, Denmark) for 30 min. Subsequently, tissue sections were incubated with the primary antibody (pancreas with an anti-insulin dilution 1:8000; Dako, A0564 and small intestine with an anti-GLP-1 dilution 1:2000; Abcam, ab26278) overnight at 4 °C. After a rinse with TBS, incubation with a HRP secondary antibody (dilution 1:100, P0141 Dako) or Envision complex (Dako) was carried out. Finally, immunostaining was developed with diaminobenzidine, lightly counterstained with hematoxylin, and mounted in distyrene plasticizer xylene. Tissues expressing different levels of antigen were included in each immunohistochemical run to control variation between experiments. Positive and negative controls were included in each experiment. Negative controls were done, leaving out the primary antibody. As positive control, we used tissue previously shown to express the antigen of interest.

The liver slides were digitized using a histology slide scanner Aperio CS, running under the Scan Scope Console software (v.10.2.0.2352, Leica Biosystems) and further analysed using Fiji software. In H&E stained liver sections, we analysed steatosis (expressed as a percentage of the area occupied by lipid droplets) in ten random images (20x) for each animal (n = 8 for each group). In colon sections, we measured the percentage of GLP-1 positive cells per total cells in ten random fields (20x) for each animal (n = 4-7 for each group). In the adipose tissue sections, we evaluated adipocytes area and diameter with the Adiposoft software. We analysed digitalized images of H&E stained adipose sections for each animal (n = 8 for each group).

### 1.5. Quantitative Real-Time PCR (RT-qPCR)

Frozen (-80 °C) liver and adipose tissues were used for total RNA extraction using RNeasy Mini Kit (Qiagen) according to the manufacturer's instructions. The purity and concentration were determined by spectrophotometry using Nanodrop One (Thermo Scientific). Then, cDNA synthesis (RT) was performed using SuperscriptTM IV VILOTMRT Premix with enzDNaseTM (Invitrogen) following the manufacturer's instructions. The mRNA expression levels of lipid metabolism-related genes *(Srebp, Fasn, Ppara, Pparγ, Acox, Cpt1* and *CD36*) were analysed and further normalized using *H3* or *Gapdh* as a house-keeping gene for liver or adipose tissue respectively. Quantitative real-time PCRs (qPCR) were performed with the IQ SYBR Green Supermix (Bio-Rad) in a CFX96 Real-Time System (Bio-Rad). Data is expressed as the relative mRNA normalized to *Gapdh* and analysed according to the comparative cycle threshold method (2^{-ΔΔ CT}). Primer sequences for the targeted mouse genes and sources are shown in Table 2.

**Table 2. Primer sequences**

| **Target gene** | **Forward/ Reverse** | **Primer sequence (5' to 3')** | **SEQ ID NO** |
|---|---|---|---|
| ***Acox*** | F | CTATGGGATCAGCCAGAAAG | SEQ ID NO: 1 |
| | R | AGTCAAAGGCATCCACCAA | SEQ ID NO: 2 |
| ***CD36*** | F | CACAGCTGCCTTCTGAAATGTGTGG | SEQ ID NO: 3 |
| | R | TTTCTACGTGGCCCGGTTCTAATTC | SEQ ID NO: 4 |
| ***Cpt1*** | F | TCTAGGCAATGCCGTTCAC | SEQ ID NO: 5 |
| | R | GAGCACATGGGCACCATAC | SEQ ID NO: 6 |
| ***Fasn*** | F | AGCCATGGAGGAGGTGGTGAT | SEQ ID NO: 7 |
| | R | GTGTGCCTGCTTGGGGTGGAC | SEQ ID NO: 8 |
| ***Pparα*** | F | ACAAGGCCTCAGGGTACCA | SEQ ID NO: 11 |
| | R | GCCGAAAGAAGCCCTTACAG | SEQ ID NO: 12 |
| ***Pparγ*** | F | GCTGTTATGGGTGAAACTCTG | SEQ ID NO: 13 |
| | R | GAATAATAAGGTGGAGATGCAGG | SEQ ID NO: 14 |
| ***Srepb*** | F | CACTTCATCAAGGCAGACTC | SEQ ID NO: 17 |
| | R | CGGTAGCGCTTCTCAATGGC | SEQ ID NO: 18 |

### 1.6. Statistical analysis

Data are presented as mean ± standard deviation (SD). All statistical procedures were performed using GraphPad Prism 8.0.1 software. Statistical significance was set at p < 0.05. * denotes p < 0.05, ** denotes p < 0.01, *** denotes p < 0.001.

Data were analysed by using one-way analysis of variance (ANOVA) followed by Tukey's post-hoc test or the Kruskal-Wallis test followed by the Dunn's test for multiple comparisons, as appropriate.

### 2. Results

### 2.1. The postbiotic Pediococcus acidilactici CECT 9879 pA1c^{®}HI prevents Body Weight gain and improved obesity-related parameters

After 15 weeks with High Fat Diet like an hypercaloric conditions in obesity, the animals supplemented with the postbiotic gained 40% less weight than the non-supplemented animals (*p* < 0.05) (Figure 8A). Moreover, Figure 8B shows that the total weight gained (%) after 15 weeks, was significantly lower (*p* < 0.05) with pA1c^{®}HI vs. the Co group, evidencing the strong anti-obesogenic effect of pA1c^{®}HI. Surprisingly, statistically significant differences were also observed between the pA1c^{®}HI and pA1c^{®} groups (*p* < 0.05) (Figure 8A and 8B), emphasising that pA1c^{®}HI is more effective in reducing total BW gain and modulating the lipid metabolism than pA1c^{®}.

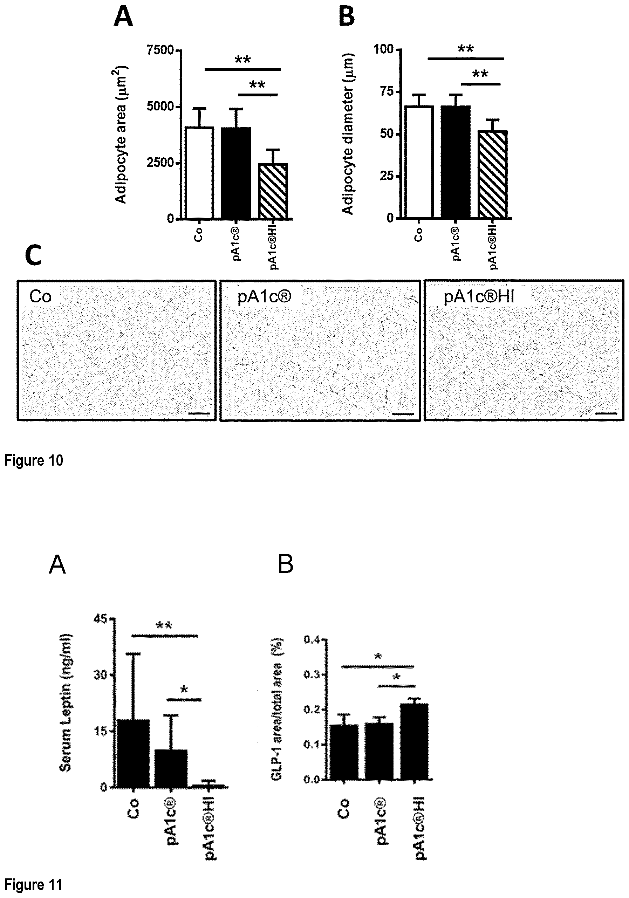

On the other hand, mice ate significantly less amount of feed when the postbiotic pA1c^{®}HI was supplemented in the diet vs the Co group (*p* < 0.05) (Figure 9A), suggesting that surprisingly, pA1c^{®}HI could affect the satiety centre of the animal by activating it. Besides, as shown in Figure 9B, we found differences in food efficiency (described as grams of weight gain /grams of food intake) between the control and postbiotic animals (Co vs pA1c^{®}HI, *p* < 0.01). Therefore, this confirmed that BW control was related to postbiotic administration rather than variations in food intake. Moreover, we found that the administration of the postbiotic pA1c^{®}HI slowed the increase in adipocyte size (*p* < 0.01) (Figures 10A-10B), and that this was directly related to a lower amount of adipose tissue in the pA1c^{®}HI group (Figure 10C) and directly correlated to the lower BW gain, demonstrating once again the adipose tissue-reducing activity of pA1c^{®}HI.

### 2.2. pA1cOHl controlled leptin regulation in HFD-fed mice

Leptin, a hormone produced by fat cells, is pivotal in the development of obesity.

It regulates appetite and energy balance by sending signals to the brain about the amount of fat stored. In obesity, elevated leptin levels may indicate resistance, reducing the response to satiety signals. This resistance contributes to increased intake and excess fat storage, thereby contributing to the perpetuation of obesity.

Serum leptin levels of the pA1c^{®}HI group were strongly inhibited when comparing to control group (*p* < 0.01) and between pA1c^{®}HI group and pA1c^{®} group (*p* < 0.05). This confirmed that the postbiotic supplementation affect satiety and the food intake and prevent the development of obesity and demonstrating that pA1c^{®}HI is more effective than pA1c^{®} in the management and treatment of obesity (Figure 11A).

On the other hand, the immunohistochemical quantification of colonic GLP-1 revealed an increased GLP-1⁺ area in the pA1c^{®}HI group with respect to the other two groups (*p* < 0.05) (Figure 11B). This finding is particularly noteworthy, as enhanced GLP-1 release is associated with beneficial effects in managing obesity. GLP-1 is known to play a critical role in regulating appetite and satiety, potentially leading to more controlled food intake and weight loss. This unexpected increase in GLP-1 expression may provide insight into the mechanism of action of pA1c^{®}HI, supporting its potential as a novel therapeutic approach for obesity management by modulating GLP-1 levels.

### 2.3. pA1cOHl modulated hepatic lipid metabolism and had an beneficial effects on adipose tissue lipid metabolism gene expression

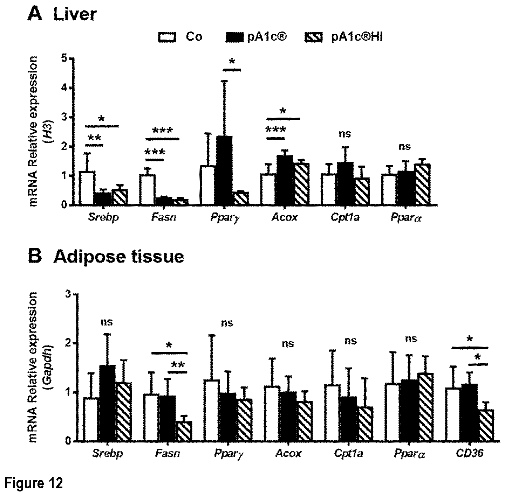

We analysed the gene expression of several lipid metabolism markers in both in liver and adipose tissue. In terms of liver analysis (Figure 12A), we can highlight that the expression of *de novo* lipogenesis (DNL) markers Srebp and *Fasn* was downregulated in the pA1c^{®}HI group (p < 0.05) when compared to the Co group, evidencing the implication of pA1c^{®}HI in the *de novo* lipogenesis process, giving us more information about the surrounded mechanism of action.

*Ppary,* a lipid uptake and adipogenic marker, significant differences were found between the pA1c^{®}HI and pA1c^{®} groups (*p* < 0.05). Regarding beta-oxidation pathway markers, we found differences for the peroxisomal fatty acid degradation gene Acox, which expression was higher in pA1c^{®}HI groups compared to the Co group (*p* < 0.05) suggesting a beta-oxidation activation in the peroxisome. In conclusion, the postbiotic had metabolic effects in the liver, decreasing DNL and increasing the catabolic peroxisomal beta-oxidation pathway.

On the other hand, in the adipose tissue (Figure 12B), DNL marker *Fasn* decreased only in the pA1c^{®}HI group in comparison with co and pA1c^{®} (pA1c^{®}HI vs. Co, *p* < 0.05; and pA1c^{®}HI vs. pA1c^{®}, *p* < 0.01), and no differences were found for Srebp or the lipid uptake marker *Pparγ(p* > 0.05). Unlike in the liver, no significant differences were found for the beta-oxidation pathway markers *(Acox, Cpt1* or *Ppara (p* > 0.05) but we did find significant differences for CD36, being its expression lower in the pA1c^{®}HI group compared to the Co or pA1c^{®} groups (*p* < 0.05). In summary, since CD36 is involved in the import of fatty acids into cells, the administration of the postbiotic decreased the store of fatty acids in adipose tissue cells and DNL.

In conclusion, the results surprisingly demonstrate that the postbiotic pA1c^{®}HI is more effective than the probiotic pA1c^{®} in modulating lipid metabolism, with notable effects in both the liver and adipose tissue. While both the postbiotic and the probiotic exhibited similar metabolic effects in the liver, reducing de novo lipogenesis (DNL) and enhancing beta-oxidation, the postbiotic showed a more significant impact in adipose tissue. Specifically, pA1c^{®}HI significantly decreased the DNL marker Fasn and lowered the expression of CD36, which is involved in fatty acid uptake into cells, thereby reducing fatty acid storage in adipose tissue. Additionally, the increased GLP-1+ area observed in the pA1c^{®}HI group may further explain the postbiotic's enhanced fat-reducing effects. The potential interaction between GLP-1 and key genes such as PPARγ, involved in lipid uptake and adipogenesis, suggests that pA1c^{®}HI may influence both fat storage and appetite regulation, making it a potent alternative for obesity management. These findings highlight the postbiotic's ability to not only modulate lipid metabolism through genes like *Fasn, CD36,* and *PPARγ* but also to promote beneficial gut hormone regulation through increased GLP-1 release, offering a more comprehensive and effective strategy for reducing fat accumulation.

### 2.4. pA1cOHl improved gut microbiota composition

Comparisons between groups were performed to further analyse the differences in relative abundance. Surprisingly, pA1c^{®}HI consumption increased the abundance of the genus *Atopostipes (p* < 0.05) while decreased two others genera *(Clostridium XVIII* and *Fusibacter)(p* < 0.001) in comparison with Co animals. Literature review showed that the genus *Fusibacter* is abundant in the intestinal microbiota of animals with greater body weight. This bacterium presents thiosulfate- and sulfur-reducing properties, which have been associated to HFD consumption. Surprisingly, it is possible that pA1c^{®}HI prevented the HFD-associated increase in *Fusibacter* in the animals supplemented with the postbiotic.

Additionally, *Clostridium cluster XVIII* is known to be more abundant in individuals with obesity compared to controls. Notably, treatments that reduce the abundance of *Clostridium cluster XVIII* have been positively correlated with improvements in body weight and body mass index in obese patients. Consistent with this, our study found that the group with the lowest abundance of *Clostridium cluster XVIII* (pA1c^{®}HI) also exhibited the best body composition regulation and weight loss.

In conclusion, pA1c^{®}HI supplementation led to an increase in beneficial genera such as *Atopostipes,* while reducing the abundance of *Fusibacter* and *Clostridium cluster XVIII,* which are associated with obesity. This shift in microbiota composition correlated with improved body composition and weight loss, highlighting the potential of pA1c^{®}HI for obesity management.

### Example 4. Clinical study

### 1. Material and Methods

### 1.1. Study Design

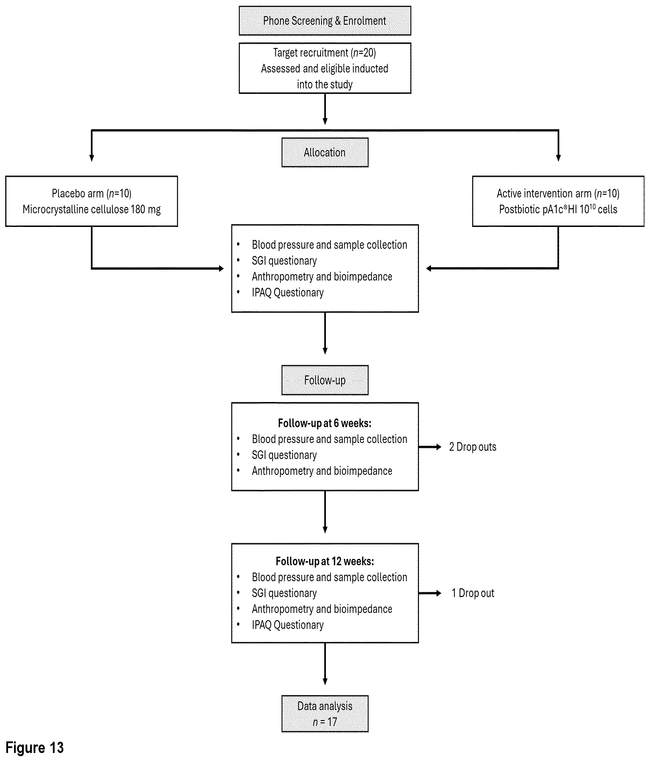

This pilot study was a single site, randomized, double blind, placebo-controlled nutritional intervention with two parallel study groups. The study was aimed at obese men and women aged 18 to 70 years. The intervention was carried out in the facilities of the Hospital Universitario del Vinalopo. 17-adults with obesity were randomized to take either a postbiotic capsule (*n* = 10) or placebo capsule (*n* = 10) for 12 weeks. Participants' progression through the intervention is presented in Fig 13. Participants were randomized to the postbiotic or placebo group without stratification using computer-generated random numbers. Both participants and study investigators were blinded to intervention allocation.

In order to was eligible to participate in the study, interested parties must met the following inclusion criteria: Men and women aged between 18 and 70 years, Body Mass Index (BMI) of over 30 kg/m², subjects must were in general physical and psychological condition as assessed by the investigator in accordance with the aim of the study, subjects must be able to understand and be willing to sign the informed consent form and must comply with all study procedures and requirements.

The product under study was a postbiotic formulation pA1c^{®}HI *Pediococcus acidilactici* CECT 9879, or alternatively the placebo product. The postbiotic formulation contained Microcrystalline cellulose, Magnesium stearate, Corn-starch, and 10<10> heat-killed *Pediococcus acidilactici* CECT 9879 cells in a vegetable transparent capsule. The placebo consisted on the same formulation without heat-killed *Pediococcus acidilactici* CECT 9879 The presentation of the product was in capsules. Consumption was scheduled to be one capsule per day (1×10¹⁰ total cells/day), at breakfast. In order to assess compliance, each participant was required to complete a product intake diary and indicate if any related adverse events or occurrences occur.

The main variables were: Anthropometric variables (weight and BMI) and body composition (fat mass, muscle mass and visceral fat) between the intervention and placebo groups, between baseline and 3 months. The secondary variables were the evolution of dietary and physical activity habits and assess adherence to intervention and adverse events. Anthropometric variables and body composition were measured with scales, measuring rod and tape measure are available, as well as bioimpedance equipment (Tanita SC-330-S) for the determination of body composition. The protocols described in Schroder H, Covas MI, Marrugat J, Vila J, Pena A, Alcántara M, Masiá R. Use of a three-day estimated food record, a 72-hour recall and a food-frequency questionnaire for dietary assessment in a Mediterranean Spanish population, Clin Nutr 2001 Oct;20(5):429-37, doi: 10.1054/clnu.2001.0460. PMID: 11534938 and Craig, C. L., et al. (2003). "International physical activity questionnaire: 12-country reliability and validity." Med Sci Sports Exerc 35: 1381-95 were used to analyze dietary and physical activity habits.

The statistical analysis of the data consisted of a presentation of the main variables, describing and comparing the baseline characteristics of the initial population between the two branches of the study.

Differences between groups were studied by Student's t-test for independent samples for normally distributed variables, or by the Mann-Whitney U-test if the variables followed a non-normal distribution. Statistical treatment of the data was performed with the GraphPad Prism v.9 or similar software package.

### 2. RESULTS

From the 20 subjects assessed for eligibility, finally 17 of them (85%) received allocated intervention in either placebo (n = 7) or the postbiotic (n = 10) groups. No differences (p > 0.05) in baseline characteristics were observed among intervention groups (Table 3) except for lean mass (p = 0.007).

**Table 3. Baseline characteristics of the variables of study of the participants. Data is expressed as mean ± standard deviation. ^{##}p < 0.01 for inter-intervention comparisons (vs Placebo).**

| | **Placebo (*n* = 7)** | **Postbiotic (*n* = 10)** | **p value** |
|---|---|---|---|
| **Age** | 46.9±5.2 | 43.4±13.4 | 0.53 |
| **Weight (Kg)** | 118.0±25.0 | 104.2±10.8 | 0.13 |
| **BMI** | 39.7±5.3 | 41.1±3.6 | 0.54 |
| **Waist circumference (cm)** | 127.4±14.7 | 118.5±7.8 | 0.12 |
| **Fat mass (Kg)** | 53.9±14.4 | 56.4±8.2 | 0.64 |
| **Lean mass (Kg)** | 28.6±7.4 | 21.2±1.8 | 0.007^{##} |
| **Visceral fat (Kg)** | 16.9±7.1 | 11.9±2.8 | 0.06 |

Moreover, all volunteers underwent the EAT-26 (eating attitudes test) questionnaire (a self-administered scale used to assess the risk of eating disorders), the IPAQ (International Physical Activity Questionnaire) questionnaire (is a tool designed to quantitatively assess a person's level of physical activity over the past seven days) and the GIS (Gastrointestinal symptoms) questionnaire (quantitatively assess a person's gastrointestinal discomfort). Diet, physical activity and gastrointestinal symptoms at the baseline were similar among groups (Table 4).

**Table 4. Baseline values of the EAT-26, IPAQ and GIS questionnaires. Data is expressed as mean ± standard deviation.**

| | **Placebo (*n* = 7)** | **Postbiotic (*n* = 10)** | **p value** |
|---|---|---|---|
| **EAT-26** | 13.1±6.5 | 13.2±5.7 | 0.984 |
| **IPAQ** | 1.7±0.8 | 1.5±0.5 | 0.499 |
| **GIS** | 28.3±10.7 | 31.5±13.4 | 0.606 |

### The postbiotic pA1cOHl reduced total weight and BMI, and improved body composition and gastrointestinal sympstoms in subjects with obesity

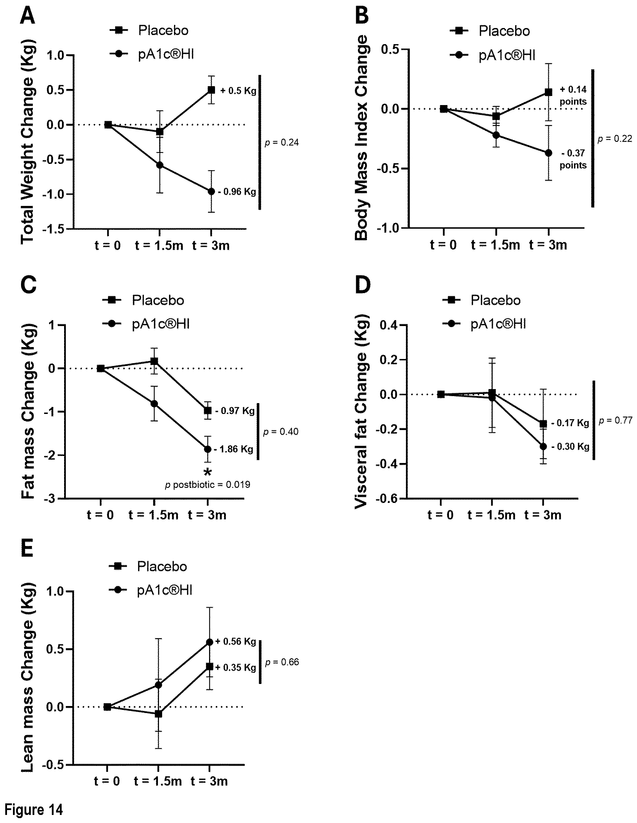

The changes at the end of the study of the main variables of the clinical trial and of the most relevant anthropometrical and biochemical parameters are shown in Figure 14. The rest of the variations observed between the two groups are shown in Table 5. This table provides a comprehensive summary of the additional parameters assessed in the study, allowing a more complete view of the results obtained.

**Table 5. Changes in biomarkers related to anthropometric measurements, glucose metabolism and lipid metabolism at baseline and at the end of the intervention in both study groups. Data is expressed as mean ± standard deviation. *p < 0.05 for placebo or postbiotic intra-intervention comparisons. ^{#}p < 0.05 for inter-intervention comparisons (vs Placebo).**

| | **Placebo (*n* = 7)** | | **Postbiotic (*n* = 10)** | | **Δpostbiotic-placebo** | |
|---|---|---|---|---|---|---|
| | Δfinal-initial | *p* value | Δfinal-initial | *p* value | Δpostbiotic-placebo | p value |
| **Weight (Kg)** | 0.5±3.1 | 0.68 | -1.0±1.9 | 0.14 | -1.5 | 0.24 |
| **BMI** | 0.1±0.9 | 0.69 | -0.4±0.7 | 0.15 | -0.5 | 0.22 |
| **Waist circumference (cm)** | -2.0 ±5.9 | 0.40 | -0.1±2.2 | 0.89 | 1.9 | 0.36 |
| **Fat mass (Kg)** | -1.0±1.8 | 0.25 | -1.9±2.1 | 0.019* | -0.9 | 0.40 |
| **Lean mass (Kg)** | 0.4±0.4 | 0.11 | 0.6±1.1 | 0.13 | 0.2 | 0.66 |
| **Visceral fat (Kg)** | -0.2±1.2 | 0.74 | -0.3±0.7 | 0.19 | -0.1 | 0.77 |

After 3 months, the intervention with pA1c^{®}HI led to clinically relevant improvements in anthropometric measurements, including reductions in total body weight (-1.46 kg) and BMI (-0.51 points) in comparison with placebo (Figure 14). Additionally, the postbiotic supplementation also improved body composition by reducing fat mass (-890 g), and visceral fat (-130 g), alongside an increase in lean mass (+210 g) in comparison with the placebo group (Fig 14C, 14D, 14E), evidencing that pA1c^{®}HI offers potential benefits for weight management and body composition enhancement in individuals with obesity, even after just three months of intervention.

In relation to physical activity, it was shown that all volunteers, regardless of whether they were in the placebo or postbiotic group, maintained their baseline levels of physical activity. In addition, the probiotic intervention was found to statistically significantly reduce gastrointestinal symptoms (p = 0.045) by 30.8% or 9.7 points (Table 6).

**Table 6. Changes in the IPAQ and GIS questionnaires at the end of the intervention in both study groups. Data is expressed as mean ± standard deviation.**

| | **Placebo (*n* = 7)** | | **Postbiotic (*n* = 10)** | | **Δpostbiotic-placebo** | |
|---|---|---|---|---|---|---|
| | Δfinal-initial | *p* value | Δfinal-initial | *p* value | Δpostbiotic-placebo | p value |
| **IPAQ** | 0.0±0.0 | 0.99 | -0.1±0.0 | 0.97 | -0.1±0.0 | 0.99 |
| **GIS** | -3.0±9.2 | 0.42 | -9.7±13.2 | 0.045* | -6.7±11.2 | 0.26 |

In conclusion, after 3 months of intervention with pA1c^{®}HI, clinically relevant improvements were observed in anthropometric and biochemical parameters, including reductions in body weight, BMI, fat mass, and visceral fat, as well as an increase in lean mass. These changes were more pronounced in the postbiotic group compared to the placebo group, highlighting the potential of pA1c^{®}HI for weight management and body composition enhancement in individuals with obesity. Additionally, the postbiotic supplementation led to a significant reduction in gastrointestinal symptoms, further demonstrating its beneficial effects. These results suggest that the observed improvements can be attributed primarily to the postbiotic intervention, with diet and exercise controlled throughout the study.

### INTEGRATIVE MECHANISIM OF ACTION

The postbiotic pA1c^{®}HI demonstrates a novel and surprisingly effective mechanism of action in reducing fat accumulation and improving metabolic parameters, making it a promising candidate for obesity management. In C. *elegans,* pA1c^{®}HI significantly reduces fat storage by modulating key genes involved in lipid metabolism. Specifically, it upregulates genes related to fatty acid oxidation, such as *cpt-2* and acox-1, promoting peroxisomal and mitochondrial beta-oxidation pathways. This action enhances the breakdown of fatty acids, leading to a reduction in fat accumulation. Additionally, pA1c^{®}HI decreases the expression of *fat-5,* a gene involved in de novo lipogenesis, which further supports its fat-reducing effects. These findings highlight a surprising and potent mechanism of action through which pA1c^{®}HI effectively reduces fat storage.

What makes pA1c^{®}HI particularly remarkable is its stability and effectiveness under a variety of conditions. Unlike traditional probiotics, which often lose efficacy when exposed to high temperatures or harsh environments, pA1c^{®}HI remains active even after heat treatment. It has shown remarkable fat-reducing activity after exposure to temperatures as high as 135°C, ensuring its potential for inclusion in a wide range of food products and dietary supplements. This exceptional heat tolerance and resilience offer a clear advantage over probiotics, which require strict storage and handling conditions to maintain viability.

Moreover, we saw that in *in vivo* rodent studies, pA1c^{®}HI also shows impressive results in reducing total weight and weight gain by modulating key genes involved in lipid metabolism, including *Pparγ* and CD36. The postbiotic significantly reduces the expression of *CD36,* a gene involved in the uptake of fatty acids into adipocytes, which may explain its effect on decreasing fat storage. At the same time, it modulates *Pparγ,* a key regulator of adipogenesis, further contributing to its fat-reducing effects. Additionally, pA1c^{®}HI stimulates GLP-1 secretion, a hormone that regulates appetite and satiety, which may help control food intake and weight. These genetic alterations, combined with its ability to improve the gut microbiota composition by decreasing obesity-related bacteria, highlight the multifactorial nature of pA1c^{®}HI's action in obesity management.

Furthermore, we demonstrated in a clinical trial, that supplementation with pA1c^{®}HI led to clinically relevant reductions in total body weight, body mass index (BMI), and fat mass after just three months, along with improvements in gastrointestinal symptoms in obese population.

Overall, pA1c^{®}HI's multifaceted mechanisms: modulating lipid metabolism, increasing GLP-1 secretion, promoting beneficial microbiota, and remaining stable under varying conditions; make it a highly innovative and effective postbiotic. Its surprising efficacy, even under extreme conditions, and its potential to reduce fat accumulation, improve body composition, and regulate metabolism, position it as a powerful natural alternative for addressing obesity. Moreover, pA1c^{®}HI demonstrates a synergistic effect when combined with certain micronutrients, such as chromium picolinate and zinc. In the case of chromium picolinate, the combination with pA1c^{®}HI resulted in a significant reduction in fat accumulation, with effects comparable to the anti-obesity drug Orlistat. Similarly, zinc supplementation enhanced the fat-reducing effects of pA1c^{®}HI, further potentiating its action and demonstrating that micronutrients can work synergistically with the postbiotic to improve metabolic outcomes. These findings suggest that the postbiotic's efficacy can be enhanced when used in combination with specific micronutrients, offering a potential avenue for more effective obesity treatments.

### Citation List

WO2021123355
Sambrook, J. and Russell, DW "Molecular Cloning: A Laboratory Manual", Chapter 13, "Mutagenesis", Cold Spring Harbor, 3rd Ed., 2001
Fan et al. Inactivation of Gram-Positive Bacteria by Novel Phenolic Branched-Chain Fatty Acids. J Food Prot, 2017, vol 80, issue 1, p. 6-14. doi: 10.4315/0362-028X.JFP-16-080*.*
Levinson et al, Production of potent inactivated vaccines with ultraviolet irradiation: ii. an abbreviated preliminary report on sterilization of bacteria and immunization with rabies and St. Louis encephalitis vaccines. JAMA 1944; 125(8): 531-532. doi:10.1001/jama.1944.02850260005002*.*
Hankaniemi et al, Formalin treatment increases the stability and immunogenicity of coxsackievirus B1 VLP vaccine. Antiviral Research 2019, Volume 171, 104595.
EUROPEAN PHARMACOPOEIA 6.0. (2008). 2.9.3. Dissolution test for solid dosage forms, pp. 266-275*.*
Wang, Q.; Garrity, G.M.; Tiedje, J.M.; Cole, J.R. Naïve Bayesian classifier for rapid assignment of rRNA sequences into the new bacterial taxonomy. Appl. Environ. Microbiol. 2007, 73, 5261-5267.
Yue Y, Li S, Shen P, Park Y. Caenorhabditis elegans as a model for obesity research. Curr Res Food Sci. 2021 Oct 1;4:692-697. doi: 10. 10161jcrfs.2021.09.008*.*
Escorcia W., Ruter D.L., Nhan J., Curran S.P. Quantification of Lipid Abundance and Evaluation of Lipid Distribution in Caenorhabditis elegans by Nile Red and Oil Red O Staining. J. Vis. Exp. 2018;2018:57352. doi: 10.3791/57352.
Xu A, Zhang Z, Ko SH, Fisher AL, Liu Z, Chen L. Microtubule regulators act in the nervous system to modulate fat metabolism and longevity through DAF-16 in C. elegans. Aging Cell. 2019 Apr;18(2):e12884. doi: 10. 1111/acel. 12884. Epub 2019 Jan 14*.*
Buranasin P, Mizutani K, Iwasaki K, Pawaputanon Na Mahasarakham C, Kido D, et al. (2018) High glucose-induced oxidative stress impairs proliferation and migration of human gingival fibroblasts. PLOS ONE 13(8): e0201855. https://doi.org/10.1371/íournal.pone.0201855
Schröder H, Covas MI, Marrugat J, Vila J, Pena A, Alcántara M, Masiá R. Use of a three-day estimated food record, a 72-hour recall and a food-frequency questionnaire for dietary assessment in a Mediterranean Spanish population. Clin Nutr. 2001 Oct20(5):429-37, doi: 10.1054/clnu.2001.0460. PMID: 11534938
Craig, C. L., et al. (2003). "International physical activity questionnaire: 12-country reliability and validity." Med Sci Sports Exerc 35: 1381-95

## Claims

1. A postbiotic preparation of *Pediococcus acidilactici (P. acidilactici)* for use in preventing and/or treating obesity or overweight.

2. A postbiotic preparation of *Pediococcus acidilactici (P. acidilactici)* for use in reducing body fat, in preventing fat accumulation, in reducing appetite, in reducing food intake, or in preventing weight gain.

3. The postbiotic preparation for use according to any one of claims 1-2, comprising inanimate *P. acidilactici* cells or a lysate of *P. acidilactici.*

4. The postbiotic preparation for use according to any one of claims 1-3, wherein the *P. acidilactici* is selected from the group consisting of *P. acidilactici* deposited at the "Coleccion Española de Cultivos Tipo" (CECT) with identification reference *CECT* 9879, *P. acidilactici* deposited at the "Coleccion Española de Cultivos Tipo" (CECT) with identification reference CECT 9889, *P. acidilactici* deposited at the "Coleccion Española de Cultivos Tipo" (CECT) with identification reference CECT 9967, and combinations thereof.

5. The postbiotic preparation for use according to claim 4, comprising *P. acidilactici CECT 9879.*

6. The postbiotic preparation for use according to any one of claims 2-5, wherein the use comprises administering to an animal, in particular a human, in need thereof from 10⁵ to 10¹¹ cells of *P. acidilactici* per day per animal, in particular, from 10⁹ to 10¹¹ cells of *P. acidilactici* per day per animal, preferably around 10¹⁰ cells of *P. acidilactici* per day per animal.

7. A composition comprising a postbiotic preparation as defined in any one of claims 1-6 for use in preventing and/or treating obesity or overweight.

8. A composition comprising a postbiotic preparation as defined in any one of claims 1-6 for use in reducing body fat, in preventing fat accumulation, in reducing appetite, in reducing food intake, or in preventing weight gain.

9. The composition for use according to any one of claims 7-8, wherein the composition is an oral composition.

10. The composition for use according to the preceding claim, wherein the oral composition is a food or feed product, a food or feed ingredient, or a food or feed supplement, and optionally further comprises food or feed additives selected from the group consisting of preservatives, anti-oxidants, emulsifiers, colorants, and bulking agents.

11. The composition for use according to any one of claims 7-9, wherein the composition is a pharmaceutical or nutraceutical composition and also comprises pharmaceutically acceptable excipients and carriers, in particular further comprising a lubricating agent, and/or a bulking agent, and/or an enteric coating.

12. The composition for use according to any one of claims 7-11, further comprising zinc or chromium, in particular, wherein the chromium is chromium picolinate.

13. The postbiotic preparation for use according to any one of claims 1-6 or the composition for use according to any one of claims 7-12, when used in combination with:
(a) a further active ingredient that is effective in preventing and/or treating obesity, in reducing body fat, in preventing fat accumulation, in reducing appetite, in reducing food intake, in preventing adipogenesis in the adipose tissue, or in preventing weight gain, in particular wherein the further active ingredient is selected from the group consisting of Semaglutide, Liraglutide, GLP-1/GIP dual agonists such as tirzepatide, gastrointestinal lipase inhibitor such as Orlistat, appetite suppressants such as phentermine, topiramate, or natural appetite suppresants, Naltrexone/Bupropion, Setmelanotide, Tirzepatide, Cagrilintide (an amylin analog), Monlunabant (a CB1 receptor antagonist), and inhibitors of the sodium-glucose cotransporter type 2 (SGLT2 inhibitors) such as canagliflozin, ertugliflozin, dapagliflozin, empagliflozin, and oral formulations of semaglutide, and combinations thereof; and/or
(b) a low-calorie diet and/or regular exercise.

14. The postbiotic preparation or composition for use according to any one of claims 1-6 or the composition for use according to any one of claims 7-11, when used in combination with zinc or chromium, in particular, wherein the chromium is chromium picolinate.

15. The postbiotic preparation or composition for use according to any one of claims 13-14, wherein the postbiotic preparation or composition and the further active ingredient are administered concomitantly or separately, in any order, within a therapeutically effective interval.
